# EUROPEAN PATENT SPECIFICATION

(11) **EP 2 723 391 B1**
(45) Date of publication and mention of the grant of the patent: **13.06.2018**
(21) Application number: 12803464.2
(22) Date of filing: 14.06.2012
(51) Int. Cl.: A61K 39/395, A61K 38/17, C12Q 1/68, C12P 21/00, G01N 33/53

(54) **FIBROBLAST GROWTH FACTOR RECEPTOR INHIBITION FOR THE TREATMENT OF DISEASE**
FIBROBLASTENWACHSTUMSFAKTORREZEPTOR-INHIBITOREN ZUR BEHANDLUNG VON ERKRANKUNGEN
INHIBITION DU RÉCEPTEUR DU FACTEUR DE CROISSANCE DES FIBROBLASTES POUR LE TRAITEMENT DE MALADIES

(30) Priority: 24.06.2011 US 201161501025 P
(43) Date of publication of application: 30.04.2014
(73) Proprietor: University of Miami, Miami, FL 33136 (US)
(72) Inventor: FAUL, Christian, Key Biscayne Florida 33149 (US); WOLF, Myles, Pinecrest Florida 33156 (US)
(74) Representative: Gulde & Partner
(86) International application number: PCT/US2012/042449
(87) International publication number: WO 2012/177481

(56) References cited:
- WO-A2-2012/174476
- US-A1- 2007 274 981
- US-A1- 2010 323 954
- US-A1- 2011 076 262
- US-A1- 2011 086 032
- US-A1- 2011 150 903
- ANDREW BEENKEN ET AL: "The FGF family: biology, pathophysiology and therapy", NATURE REVIEWS DRUG DISCOVERY, vol. 8, no. 3, 1 March 2009 (2009-03-01), pages 235-253, XP055184207, ISSN: 1474-1776, DOI: 10.1038/nrd2792
- HUANG ET AL.: 'FGFR4 Prevents Hyperlipidemia and Insulin Resistance but Underlies High-Fat Diet-Induced Fatty Liver.' DIABETES vol. 56, no. 10, October 2007, pages 2501 - 2510, XP055141555
- ZHANG ET AL.: 'Serum FGF21 Levels Are Increased in Obesity and Are Independently Associated With the Metabolic Syndrome in Humans.' DIABETES vol. 57, no. 5, May 2008, pages 1246 - 1253, XP055141558

## Description

### CROSS-REFERENCE TO RELATED APPLICATIONS

This application claims the benefit of Provisional Application Serial No. 61/501,025, filed June 24, 2011, for all purposes, herein.

### FIELD

The present disclosure relates to methods of treating or preventing left ventricular hypertrophy. Still further, the present disclosure relates to methods of treating or preventing diseases (e.g., diabetes, chronic kidney disease, cardiac diseases such as left ventricular hypertrophy, etc.) by inhibiting the activation of Fibroblast Growth Factor Receptors.

### BACKGROUND

Chronic kidney disease (CKD) is a global public health problem that is estimated to affect approximately 26 million Americans and many more patients worldwide (Coresh et al., JAMA., 298(17):2038-2047, 2007). The presence of CKD increases risk of premature death, and cardiovascular disease is the leading cause at all stages of CKD (Go et al. N. Engl. J. Med., 351(13):1296-1305, 2004). Left ventricular hypertrophy (LVH) is an important mechanism of cardiovascular disease in CKD that contributes to diastolic dysfunction, congestive heart failure, arrhythmia, and sudden death (Zoccali et al., J. Am. Soc. Nephrol., 15(4):1029-1037, 2004). Compared to a prevalence of 15 - 21% in the general population (Levi et al., N. Engl. J. Med., 322(22):1561-1566, 1990), LVH affects 50 - 70% of patients during intermediate stages of CKD and up to 90% by the time they reach dialysis. Although traditional risk factors, such as chronic hypertension, contribute to high rates of LVH in CKD, the regression of LVH after kidney transplantation suggests additional, CKD-specific risk factors that remain poorly defined.

In humans, the family of fibroblast growth factors (FGF) consists of 22 proteins that regulate cell proliferation, migration, differentiation, and survival (Eswarakumar et al., Cytokine Growth Factor Rev., 16(2):139-149, 2005). FGF2 is the prototypical FGF. It is expressed by many cell types including cardiac myocytes and fibroblasts, which also express FGF receptors (FGFR) (Ornitz and Itoh Bioes says, 22(2):108-112, 2001; Detillieux et al., Cardiovasc Res., 57(1):8-19, 2003). FGF2 causes cardiac hypertrophy by inducing changes in gene expression that are similar to those caused by chronic pressure overload. This results in "pathological" LVH that is characterized by increased extracellular matrix deposition, hypertrophy and apoptosis of individual myocytes, and increased risk of congestive heart failure and death (Heineke and Molkentin, Nat. Rev. Mol. Cell. Biol., 7(8):589-600, 2006). The calcineurin-nuclear factor of activated T cells (NFAT) and mitogen-activated protein kinase (MAPK) signaling cascades are central regulators of pathological hypertrophy, which is distinct from "physiological" LVH that occurs as an appropriate adaptive response to aerobic conditioning or pregnancy. In these settings, activation of the phosphoinositide-3-kinase (PI3K)-Akt signaling stimulates growth of cardiac myocytes in the absence of excessive extracellular matrix deposition or myocyte apoptosis.

FGF23 is the most recently discovered FGF (Shimada et al., J. Clin. Endocrinol. Metab., 95(2):578-585, 2001). Unlike FGF2 and other canonical FGFs, which exert their paracrine and autocrine effects by binding heparan sulfate in the extracellular matrix, topological differences in the heparin-binding region of FGF23 enable it to avoid capture in the extracellular matrix. As a result, FGF23 functions as an endocrine hormone that regulates phosphorus metabolism through binding to FGFR and klotho, its co-receptor in the kidney and parathyroid glands. The primary physiological actions of FGF23 are to augment phosphaturia by down regulating expression of sodium-phosphate co-transporters in the renal proximal tubule, and to decrease circulating concentrations of calcitriol by inhibiting expression of parathyroid hormone and renal CYP27B1 (1-a-hydroxylase), which synthesizes calcitriol, and stimulating CYP24 (24-hydroxylase), which catabolizes calcitriol (Wolf M., J. Am. Soc. Nephrol., 21(9):1427-1435, 2010).

While compensatory increases in FGF23 levels help CKD patients to maintain normal serum phosphate levels despite even severely reduced renal function, recent prospective studies of CKD and predominantly non-CKD patients demonstrated a dose-dependent association between elevated FGF23 levels and greater risk of major cardiovascular events and mortality. A plausible explanation linking high FGF23 to greater cardiovascular risk was offered by studies of CKD and non-CKD patients in which elevated FGF23 independently associated with increased left ventricular mass and increased prevalence of LVH.

FGF23 causes LVH directly (Faul C et al., J Clin Invest 2011; 121(11):4393-408). An important component of this finding is that FGF23 was previously assumed to be dependent on klotho. The inventors have surprisingly discovered that this cardiac effect occurs via FGF receptors (FGFR) but independent of klotho, which is the co-receptor for FGF23 in the kidney and the parathyroid glands that increases the binding affinity of FGF23 to FGFR (Kuro-o M et al., Nature. 1997;390(6655):45-51). As FGFR4 is present without klotho in cardiac tissue (Hughes SE et al., J Histochem Cytochem. 1997;45(7):1005-19), it would be beneficial to inhibit the interaction between FGFRs and one or more of the endocrine FGFs, i.e., FGF19, FGF21, and FGF23, in order to prevent cardiovascular disease.

### SUMMARY

The present invention is directed to subject matter as defined in the claims.

Certain embodiments of the present disclosure pertain to a method of treating or preventing a cardiovascular disease, CKD, diabetes, etc., in a subject (e.g., human) including: inhibiting activation of FGFRs (e.g., FGFR1, FGFR2, FGFR3, FGFR4) in cardiac cells. In specific embodiments, the cardiovascular disease is hypertension, congestive heart failure, left ventricular hypertrophy, uremic cardiomyopathy, diabetic cardiomyopathy, or primary cardiomyopathy.

In such embodiments wherein inhibition of an FGFR (e.g., FGFR4) is contemplated, the method may include administering to a subject a pharmaceutical composition which results in down regulation of protein expression of the FGFR (e.g., FGFR4). Such a composition may down regulate protein expression by inhibiting translation of the FGFR, for example by inhibiting the translation of FGFR mRNA, a nucleic acid sequence which is at least partially complementary to said mRNA. Such a nucleic acid may include any complementary nucleic acid. In preferred embodiments, the nucleic acid which is at least partially complementary to mRNA of the FGFR may be an antisense RNA, a shRNA or a siRNA.

In further embodiments inhibiting an FGFR (e.g., FGFR4) may be accomplished by administering a pharmaceutical composition to a subject that results in at least partial inactivation of FGFR-mediated signaling. In such embodiments, this inactivation may be achieved by administration of an antibody which binds to the FGFR (e.g., an antibody which binds specifically to FGFR4).

Alternatively or additively, such a method may include administering to a subject an antibody which binds to one or more endocrine FGFs (one or more of FGF19, FGF21, and FGF23) which prevents interaction between the one or more endocrine FGFs (one or more of FGF19, FGF21, and FGF23); and an FGFR (e.g., FGFR4). Still further, in certain embodiments, a small molecule may be used to inhibit the signaling of FGFR or to inhibit an FGFR signaling pathway. Alternatively or additively, a pharmaceutical composition including a small molecule that inhibits the production or secretion of one or more FGFs (one or more of FGF19, FGF21, and FGF23) may be used. Additionally or additively, a pharmaceutical composition including a chimeric decoy of an FGFR which is able to bind to or interact with one or more FGFs (one or more of FGF19, FGF21, and FGF23) may be used.

In such embodiments, wherein a plurality of pharmaceutical compositions is employed in the treatment or prevention of CKD, diabetes, and/or cardiovascular disease such as hypertension, congestive heart failure or left ventricular hypertrophy, each pharmaceutical composition may be administered to a subject at the same time or at different times.

Certain other embodiments of the disclosure concern a pharmaceutical composition including an inhibitor of signaling mediated by one or more FGFs (one or more of FGF19, FGF21, and FGF23) and an inhibitor of FGFR (e.g., FGFR4) activation or signaling.

In such embodiments, wherein a pharmaceutical composition is concerned, the inhibitor of signaling mediated by one or more FGFs (one or more of FGF19, FGF21, and FGF23) may be, for example, one of: a complementary nucleic acid molecule, an antibody, a small molecule or a combination thereof.

In other embodiments wherein a pharmaceutical composition is concerned, the inhibitor of FGFR (e.g., FGFR4)- mediated activation or signaling may be, for example, one of: a complementary nucleic acid molecule, an antibody, a small molecule or a combination thereof.

Other embodiments of the disclosure concern a method of determining whether a subject having a cardiovascular disease is in need of inhibiting an interaction between at least one of: at least one endocrine FGF (FGF19, FGF21, and FGF23); and an FGFR (e.g., FGFR4), including: (a) obtaining a database of subjects without cardiovascular disease such as hypertension, congestive heart failure or left ventricular hypertrophy and with normal blood levels of the at least one endocrine FGF; (b) obtaining a blood sample from a subject having left ventricular hypertrophy; (c) determining if the blood level of the at least one endocrine FGF in the subject is elevated compared to the average level based on the database; and (d) administering a pharmaceutical formulation to inhibit the interaction between the at least one endocrine FGF and the FGFR if said blood level in the patient is elevated compared to the average level based on the database.

### BRIEF DESCRIPTION OF THE DRAWINGS

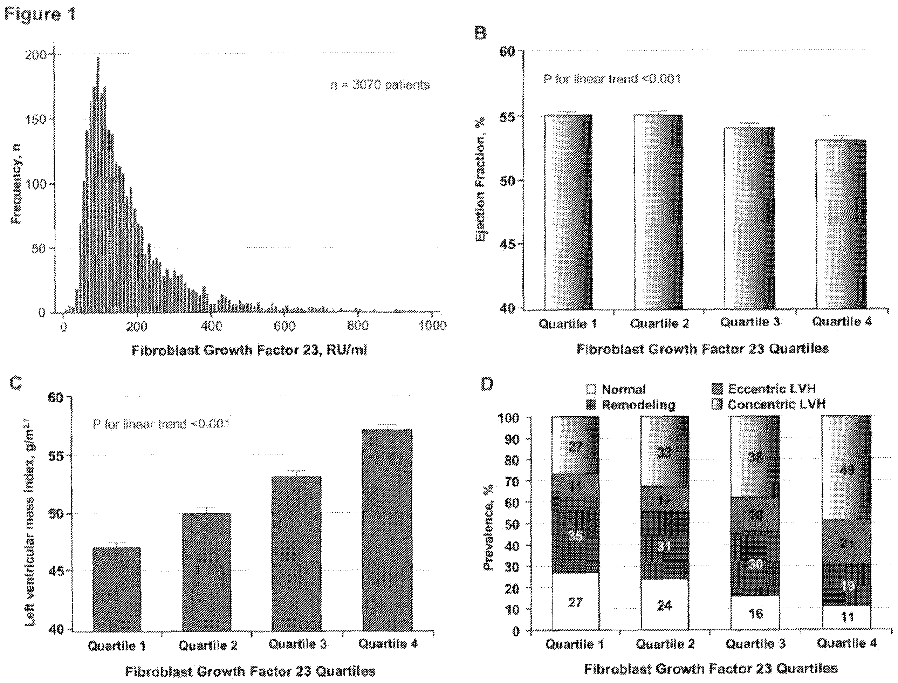

Figure 1. FGF23 is associated with LVH in patients with chronic kidney disease. (A) The distribution of FGF23 levels in baseline samples of 3044 patients who enrolled in the Chronic Renal Insufficiency Cohort and underwent echocardiography 1 year later. The median is 142 RU/ml. Fifty-eight patients with FGF23 >1000 RU/ml (range 1054 - 14319 RU/ml) who were included in the analysis are not shown here. (B) Ascending quartiles of FGF23 are associated with significantly decreased ejection fraction (p for linear trend < 0.001), but the differences between groups are modest and the mean (± standard error) ejection fraction for each quartile is normal (>50%). (C) Ascending quartiles of FGF23 are associated with significantly increased mean (± standard error) left ventricular mass index (p for linear trend < 0.001). (D) With increasing quartiles of FGF23, the prevalence of concentric (grey) and eccentric (green) LVH increases at the expense of normal left ventricular geometry (white) and left ventricular remodeling (blue) (p<0.001).

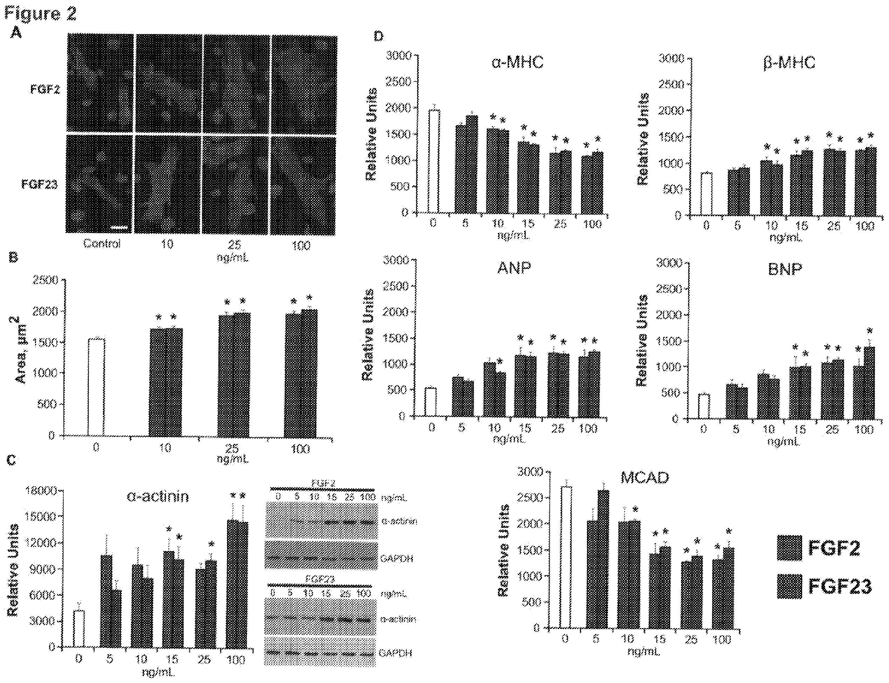

Figure 2. FGF23 increases the surface area of isolated NRVM and activates hypertrophic gene programs. (A) Surface area of isolated NRVM increase after FGF23 and FGF2 treatment as revealed by immunocytochemical analysis using antibodies to α-actinin (red). DAPI (blue) identifies nuclei. (100x magnification; scale bar = 25 µm). (B) Compared to untreated control cells (white), 48 hours of treatment with FGF23 (blue) or FGF2 (red) significantly increases cell surface area (mean ± standard error). Fifty cells per group per isolation were analyzed by morphometry (n=3 isolations of NRVM, * p<0.01 compared with untreated). (C) Compared to untreated control cells (white), FGF23 (blue) or FGF2 (red) significantly increase α-actinin protein levels normalized to GAPDH in isolated NRVM as determined by immunoblotting (mean ± standard error; n=3 isolations of NRVM, * p<0.01). (D) Compared to untreated control cells (white), FGF23 (blue) or FGF2 (red) decrease levels of a-myosin heavy chain (a-MHC) and medium chain acyl-CoA dehydrogenase (MCAD) mRNA, and increase f3-myosin heavy chain (f3-MHC), atrial natriuretic peptide (ANP) and brain natriuretic peptide (BNP) mRNA (mean ± standard error; n=3 isolations of NRVM quantified by RT-PCR normalized to GAPDH; * p<0.01 compared with untreated).

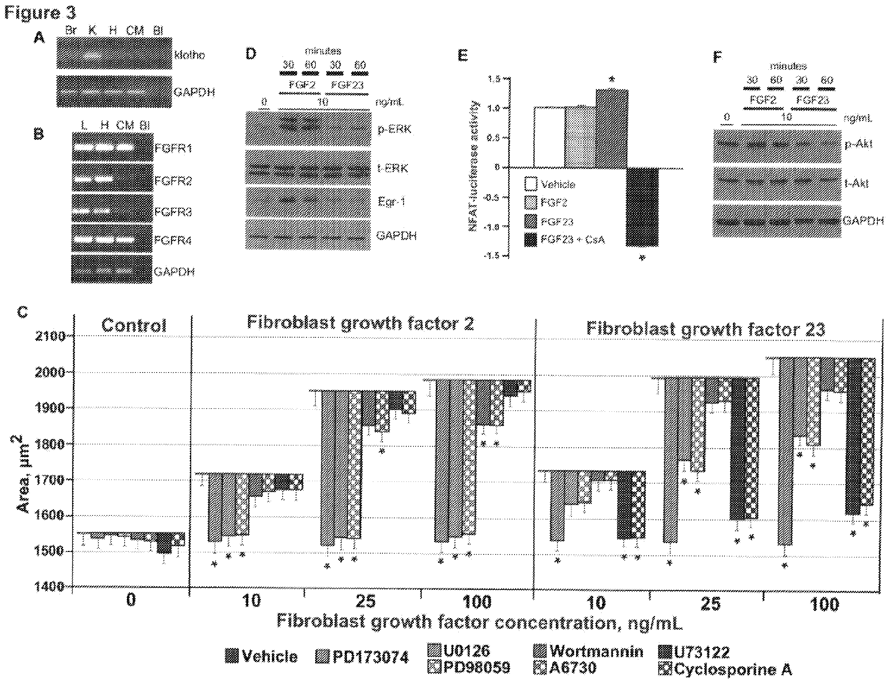

Figure 3. FGF23 and FGF2 utilize different signaling pathways to induce hypertrophy of NRVM. (A) Klotho expression is detectable in mouse brain (Br) and kidney (K) by nested RT-PCR but not in heart (H), isolated cardiac myocytes (CM), or in the absence of template (blank, B1). (B) FGFR1 - 4 are detectable in liver (L), heart (H) and isolated cardiac myocytes (CM) by RT-PCR but not in the absence of template (blank, B1). (C) Surface area of isolated NRVM after 48 hours of treatment with FGF23 or FGF2 alone and in the presence of inhibitors. The lower horizontal edge of the bars represents the mean (± standard error) surface area of the cells and the height of the bars represents the difference in surface area between cells that were treated with FGF alone and those that were co-treated with inhibitor. Inhibiting FGFR (grey) prevents any increase in cell surface area regardless of FGF concentration. ERK inhibition (orange) completely prevents FGF2-but only partially prevents FGF23-induced hypertrophy. PI3K/Akt inhibition (green) partially prevents FGF2-induced hypertrophy but has no effect on FGF23-treated cells. PLCy/calcineurin inhibition (blue) prevents FGF23-induced hypertrophy but has no effect on FGF2-induced hypertrophy. 150 cells were analyzed per condition. * p<0.01 compared with corresponding FGF concentration in the absence of inhibitor (red). (D) After 30 and 60 minutes, FGF2 stimulates a greater increase in ERK phosphorylation (p-ERK) and Egr-1 expression than FGF23 while total ERK (t-ERK) levels remain unchanged. (E) After 60 minutes, FGF23 but not FGF2 stimulates dephosphorylation of NFAT. (F) After 60 minutes, FGF2 but not FGF23 causes a modest increase in Akt phosphorylation (p-Akt) and no change in total Akt (t-Akt).

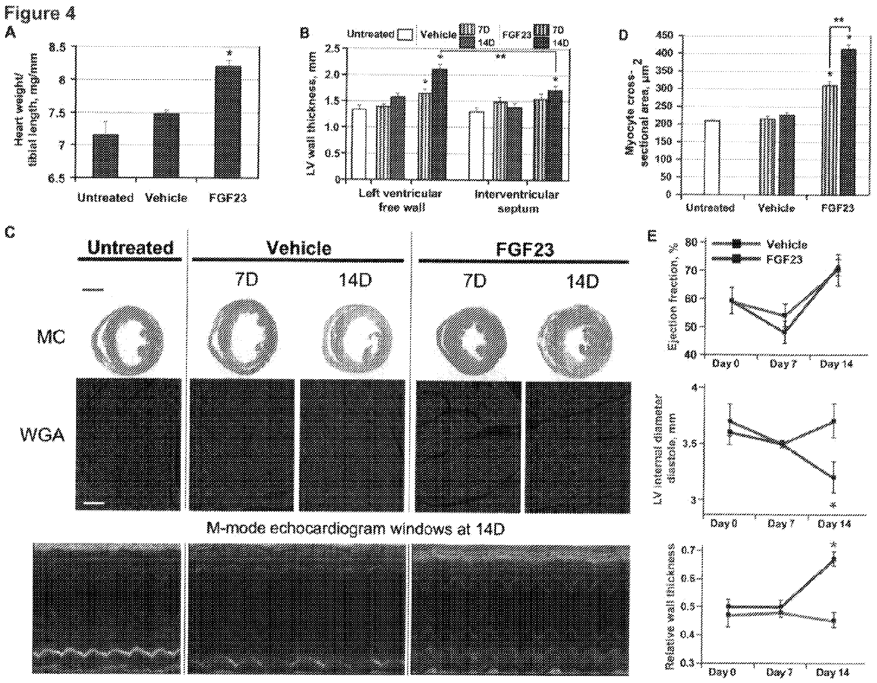

Figure 4. Intra-myocardial injection of FGF23 induces LVH in mice. (A) Intra-myocardial injection of FGF23 induces a significant increase in the ratio of heart weight to tibial length by day 14, indicating increased cardiac mass (mean ± standard error; n=3 mice per group, * p<0.01 compared to vehicle). (B) Intra-myocardial injection of FGF23 induces a significant increase in thickness of the left ventricular free wall that is detectable at day 7 and progressed by day 14. Hypertrophy is significantly more pronounced at the injection site in the free wall compared with the interventricular septum at 14 days (** P<0.01 comparing free wall to septum; mean ± standard error; n=3 mice per group, * p<0.01 compared to vehicle at corresponding date and site). (C) Representative gross pathology section from the cardiac apex, mid-chamber (MC), and base (5x magnification, scale bar = 200 µm), and wheat germ agglutinin (WGA)-stained sections from the mid-chamber free wall (63x magnification, scale bar = 50 µm) demonstrate FGF23-indcued LVH 14 days after injection (hematoxylin & eosin stain). (D) Intramyocardial injection of FGF23 induces significantly increased cross-sectional surface area of individual cardiomyocytes (*P < 0.01, compared with vehicle; **P < 0.01, comparing day 14 versus 7). (E) Echocardiography at baseline and at 1 and 2 weeks after injection of FGF23 or vehicle reveals no change in ejection fraction but significantly decreased left ventricular internal diameter in diastole and increased relative wall thickness by day 14 in the mice injected with FGF23, consistent with concentric LVH (*P < 0.01, compared with vehicle). All values are mean ± SEM; n = 3 mice per group for morphological analyses; n = 100 cells per group for WGA analysis.

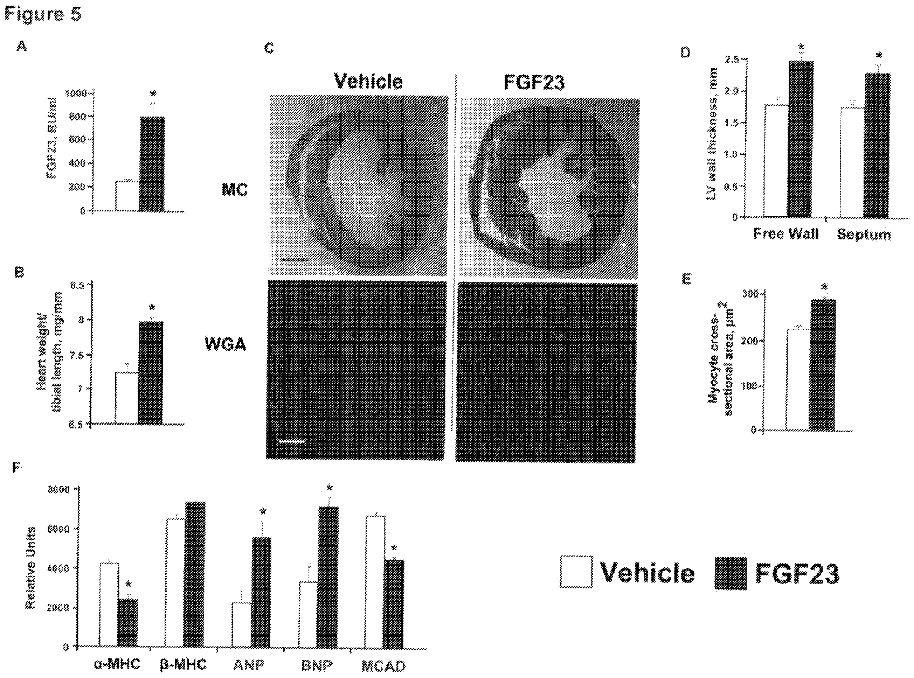

Figure 5. Intravenous injection of FGF23 results in LVH in mice. (A) Intravenous injection of FGF23 results in a significant increase in FGF23 levels (mean ± standard error; n=11 mice per group, * p<0.01 compared to vehicle). (B) Intravenous injection of FGF23 results in a significant increase in cardiac weight/tibial length (mean ± standard error; n=11 mice per group, * p<0.01 compared to vehicle). (C) Representative gross pathology (hematoxylin & eosin stain, 5x magnification, scale bar = 200 µm) and wheat germ agglutinin (WGA)-stained sections (63x magnification, scale bar = 50 µm) from the mid-chamber (MC) of the left ventricle demonstrate LVH in mice that received intravenous injections of FGF23. (D) Mice that received intravenous injections of FGF23 manifest a significant increase in left ventricular (LV) wall thickness (*P<0.01 compared to vehicle; mean ± standard error; n=5 mice per group). (E) Mice that received intravenous injections of FGF23 manifest a significant increase in cross-sectional surface area of individual cardiac myocytes (*P<0.01 compared to vehicle; mean ± standard error; n=100 cells per group). (F) Intravenous injection of FGF23 results in a significant decrease in expression of a-myosin heavy chain (a-MHC) and medium chain acyl-CoA dehydrogenase (MCAD) mRNA, and increased f3-myosin heavy chain (f3-MHC), atrial natriuretic peptide (ANP) and brain natriuretic peptide (BNP) mRNA (mean ± standard error; n=xx mice per group quantified by RT-PCR normalized to GAPDH; * p<0.01 compared to vehicle).

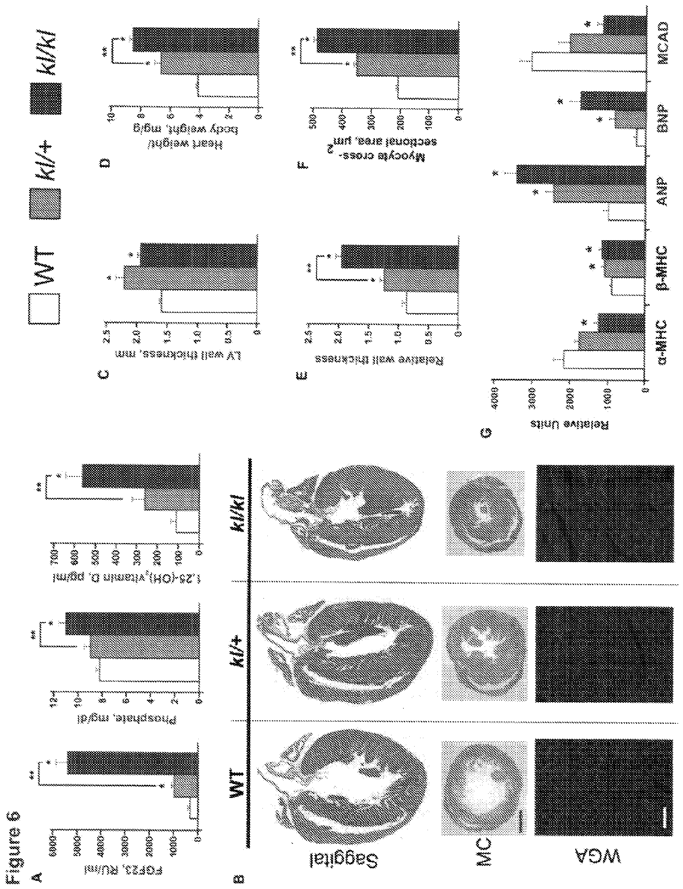

Figure 6. Klotho-ablated and klotho heterozygous mice develop LVH. (A) Compared with wild type, klotho-ablated (kl/kl) mice demonstrate significant increases in levels of FGF23, serum phosphate and 1, 25-dihydroxyvitamin D. Only FGF23 was significantly increased in klotho heterozygotes (kl/+) compared with wild type (mean ± standard error; n=6 mice per group, * p<0.01 compared to wild type; ** p<0.01 compared to kl/+). (B) Representative gross pathology of saggital and mid-chamber (MC) sections of the heart (hematoxylin & eosin stain, 5x magnification, scale bar = 200 i.tm), and wheat germ agglutinin (WGA)-stained sections from the left ventricular mid-chamber free wall (63x magnification, scale bar = 50 µm) demonstrate LVH in kl/kl and kl/+. (C) kl/kl and kl/+ manifest a significant increase in left ventricular (LV) wall thickness (*P<0.01 compared to wild type; mean ± standard error; n=6 mice per group). (D) kl/kl and kl/+ manifest significant increases in the ratio of heart weight to total body weight; (E) relative wall thickness; and (F) cross-sectional surface area of individual cardiac myocytes (*P<0.01 compared to wild type; ** p<0.01 compared to kl/+; mean ± standard error; n=6 mice per group and 100 cells per group). (G) Compared to wild type, kl/kl and kl/+ demonstrate decreased levels of a-myosin heavy chain (a-MHC) and medium chain acyl-CoA dehydrogenase (MCAD) mRNA, and increased f3-myosin heavy chain (f3-MHC), atrial natriuretic peptide (ANP) and brain natriuretic peptide (BNP) mRNA (mean ± standard error; n=3 mice per group quantified by RT-PCR normalized to GAPDH; * p<0.01 compared to wild type).

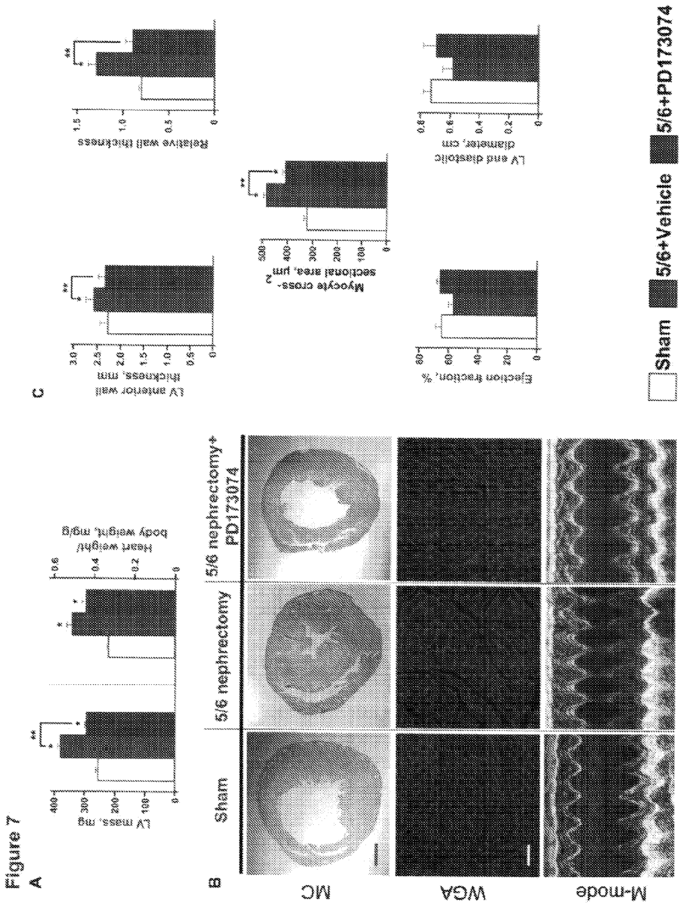

Figure 7. Pharmacological inhibition of FGFR attenuates LVH in an animal model of CKD. (A) The pan-FGFR inhibitor PD173074 attenuates the increases in left ventricular mass (by echocardiography) and cardiac weight/body weight that develop in 5/6 nephrectomized rats treated with vehicle. (B) Representative gross pathology sections, M-mode echocardiography images, and wheat germ agglutinin (WGA)-stained sections from the left ventricular mid-chamber (MC) at day 14 after 5/6 nephrectomy demonstrate that PD173074 attenuates LVH compared with vehicle. (C) PD173074 attenuates the effects of 5/6 nephrectomy to increase left ventricular (LV) anterior wall thickness and relative wall thickness (by gross pathology); to increase cross-sectional surface area of individual cardiac myocytes (by WGA-staining); and to decrease ejection fraction and LV end diastolic volume (by echocardiogram; *P < 0.05, compared with sham; **P < 0.05, compared with 5/6 nephrectomy treated with vehicle). All values are mean ± SEM (n = 6 rats per group).

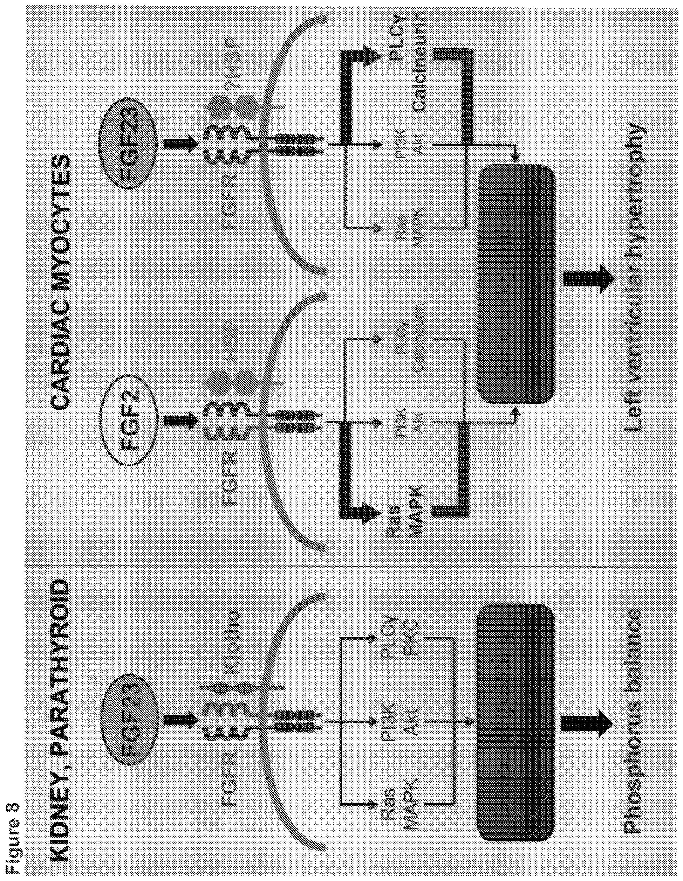

Figure 8. Schematic representation of FGF23 signaling in classic target cells and cardiac myocytes. Left: In the kidney and parathyroid glands, FGF23 signaling requires FGFR and the co-receptor klotho. FGF23-klotho binding to FGFR stimulates auto-phosphorylation of the receptor tyrosine kinase and induces signaling through three major pathways: Ras-MAPK, phosphoinositide-3-kinase (PI3K)-Akt, and PLCy-protein kinase C (PKC). FGF23 regulates phosphorus balance by altering expression of genes involved in parathyroid, vitamin D and phosphorus metabolism. Right: In cardiac myocytes, FGF2 signaling requires FGFR and heparan sulfate proteoglycans (HSP) as co-receptor, and signals primarily through the Ras-MAPK pathway. Binding of FGF23 to FGFR on cardiac myocytes stimulates auto-phosphorylation of the receptor tyrosine kinase independent of klotho, which is not expressed in cardiac myocytes, and signals primarily through the PLCy-calcineurin pathway. Whether HSP acts as co-receptor remains to be determined.

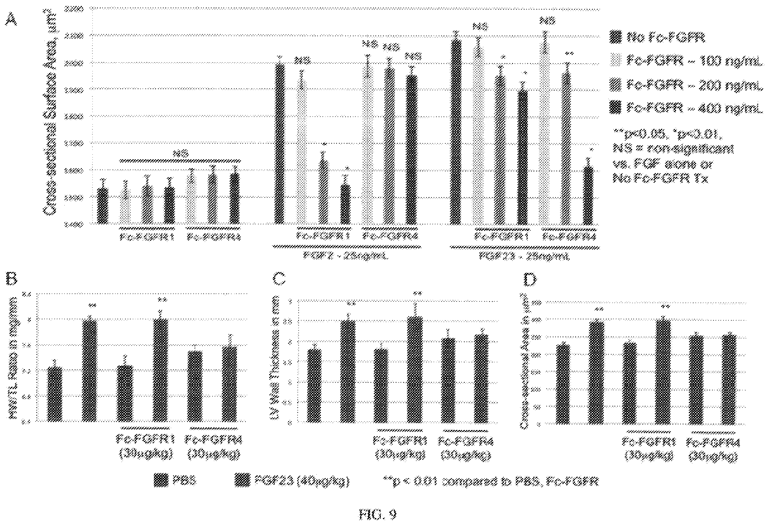

Figure 9. Series of graphs showing that inhibiting FGF23 binding to FGFR4 attenuates FGF23-induced LVH in vitro and in vivo. (A) Surface area of isolated NRVM after 48 hours of FGF treatment in the presence of increasing concentrations of decoy receptor Fc-FGFR1 or Fc-FGFR4. Neither Fc-FGFR by itself causes an increase in cell area (left panel), while FGF2 and FGF23 induce hypertrophy (green) as described in Example 1 below. Fc-FGFR1 significantly decreases area in FGF2-treated cells in a concentration-dependent manner, while Fc-FGFR4 has no effect. FGF23 induced hypertrophy is prevented in the presence of Fc-FGFR4. Fc-FGFR1 also reduces surface area in FGF23 treated cells, but at highest concentrations to a lesser extend than Fc-FGFR4 (n = 3 isolations of NRVM; 50 cells per condition). (B-D) Co-injection of Fc-FGFR4, but not of Fc-FGFR1, attenuates the FGF23-induced increase in (B) heart weight/tibial (HW/TL) length, (C) LV anterior wall thickness (determined by H&E gross pathology) and (D) cross-sectional surface area of individual cardiac myocytes (analyzed by WGA-staining). Values are mean ± SEM; n = 5 mice per group.

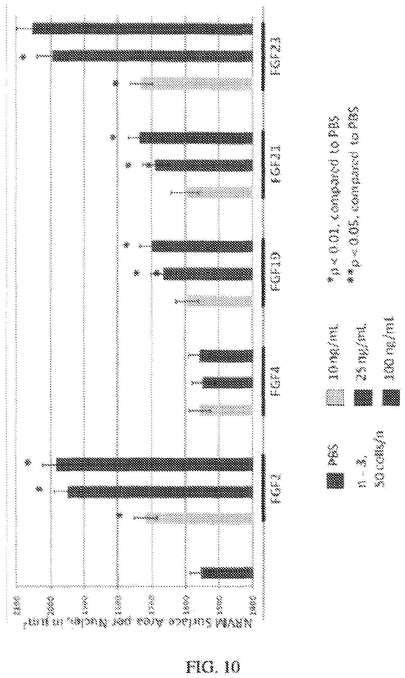

Figure 10. Series of graphs showing FGF19 and FGF21 also induce cardiac hypertrophy in vitro. Surface area of isolated NRVM after 48 hours of FGF treatment in the presence of increasing concentrations of FGFs. FGF2 and FGF23 increase cell surface area as described below in Example 1. Interestingly, also FGF19 and FGF21 cause a dose-dependent elevation of cell surface area, while FGF4 has not effect (n = 3 isolations of NRVM; 50 cells per condition).

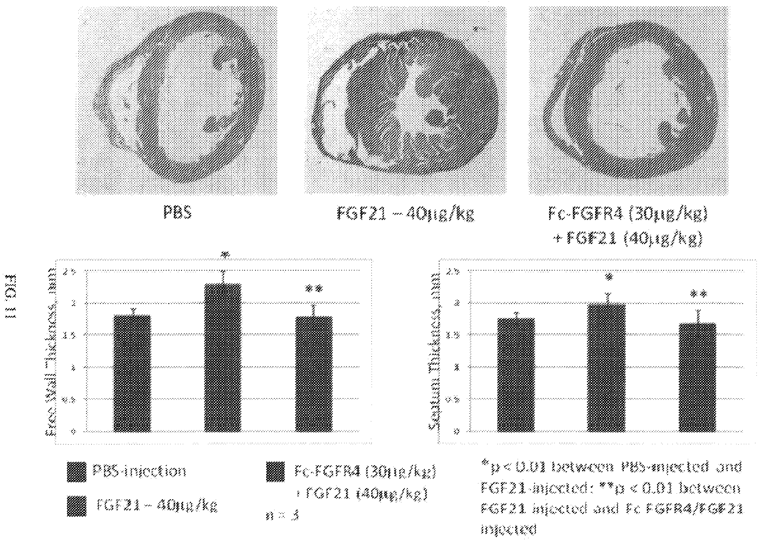

Figure 11. Series of photographs and graphs showing FGF21 induces LVH in mice in an FGFR4-dependent manner. Injections of FGF21 induce LVH in mice, and co-injections of Fc-FGFR4 attenuate this effect. (top) Representative H&E gross pathology of the heart, used for the quantification LV anterior wall and septum thickness (bottom). Values are mean ± SEM; n = 3 mice per group.

### DETAILED DESCRIPTION

### Embodiments

In certain embodiments, the disclosure relates to FGFR (e.g., FGFR4) inhibitors for the prevention, alleviation or/and treatment of left ventricular hypertrophy (LVH) and other cardiac diseases, diabetes, and CKD. In some embodiments, the compositions and methods described herein are used for attenuating or preventing LVH in individuals suffering from CKD. Further, the present disclosure relates to a diagnostic procedure wherein expression status or/and polymorphisms of an FGFR gene are determined in a patient suffering from LVH, other cardiac diseases, diabetes, and/or CKD. Based on the results of this determination and the status of the disorder to be treated a therapeutic protocol may be developed. Yet another subject of the present disclosure is a screening method. In certain other embodiments, the disclosure relates to inhibition of expression of or blocking the actions of endocrine FGFs (e.g., FGFs 19, 21 and 23) for the prevention, alleviation and/or treatment of LVH, other cardiac diseases, diabetes, and CKD. Additional examples of diseases that can be prevented, alleviated, and/or treated using the compositions and methods described herein are uremic cardiomyopathy, diabetic cardiomyopathy, and primary cardiomyopathy. In some embodiments, a composition including inhibitors to two or more FGFs (e.g., inhibitors to two or more of FGF19, FGF21, and FGF23) can be administered. Similarly, a composition including inhibitors to two or more FGFRs (e.g., FGFR1, FGFR2, FGFR3, FGFR4), can be administered. In one embodiment, a composition including an inhibitor to an endocrine FGF (e.g., an inhibitor to FGF19, FGF21, or FGF23) and an inhibitor to an FGFR (e.g., FGFR4) is administered to an individual suffering from or at risk of one or more of the diseases or conditions described herein.

### A. Inhibition of FGFR

In one embodiment of a method of treating or preventing a disease or condition associated with elevated FGF levels in a subject (e.g., human), the method includes administering to the subject a composition including a pharmaceutically acceptable carrier and an inhibitor of at least one FGFR (e.g., one FGFR, two FGFRs, three FGFRs, etc.) in an amount effective for inhibiting expression or activation of the at least one FGFR and inhibiting signaling of at least one FGF in the subject. The at least one FGF can be, for example, one or more of FGF19, FGF21, and FGF23, and the at least one FGFR can be, for example, FGFR4. In the method, the disease or condition associated with elevated FGF levels can be one or more of cardiovascular disease (e.g., LVH, hypertension, and congestive heart failure), CKD, and diabetes. The method can further include administering to the subject an inhibitor of the at least one FGF (FGF19, FGF21, and FGF23). In this embodiment, the inhibitor of the at least one FGF and the inhibitor of expression or activation of the at least one FGFR can be administered to the subject at the same time, or administered to the subject at different times.

In embodiments of the disclosure wherein inhibition of an FGFR is contemplated for the prevention, alleviation and/or treatment of LVH, the FGFR inhibitor may be a direct inhibitor or an indirect inhibitor. A direct inhibitor of an FGFR directly inhibits the FGFR, an FGFR transcript or/and the FGFR gene, thereby reducing the FGFR activity. In contrast, an indirect FGFR inhibitor does not directly inhibit the FGFR as described above, but on a target which interacts with or is regulated by the FGFR, e.g. an FGFR ligand, such as FGF23, a downstream target, such as NFAT or a protein in the MAP kinase cascade, such as Erkl or/and Erk2. Typically, FGFR and/or FGF activity is inhibited in cardiac cells in a subject (e.g., human subject).

In the present disclosure, the activity of one or more FGFRs (e.g., FGFR4) may be inhibited on the nucleic acid level, e.g. on the gene level or on the transcription level. Inhibition on the gene level may comprise a partial or complete gene inactivation, e.g. by gene disruption. Inhibition may also occur on the transcript level, e.g. by administration of anti-sense molecules, ribozymes, siRNA molecules, which may be directed against an FGFR mRNA, an FGFR ligand mRNA or/and against mRNA of a downstream target. Suitable anti-sense molecules may be selected from DNA molecules, RNA molecules and nucleic acid analogues. Ribozymes may be selected from RNA molecules and nucleic acid analogues. Small double-stranded RNA molecules capable of RNA interference (siRNA molecules) may be selected from RNA molecules and nucleic acid analogues. Preferred double-stranded siRNA molecules have a strand length of 19-25 nucleotides and optionally at least one 3'-overhang. Suitable siRNA molecules may e.g. be obtained according to Elbashir et al., 2001.

Further, the FGFR activity may be inhibited on the protein level, e.g. by administration of compounds which result in a specific FGFR inhibition. The inhibition on the protein level may comprise, for example, the application of antibodies or antibody fragments. In particular, these antibodies or antibody fragments are directed against an FGFR (e.g., FGFR4), preferably against the extracellular domain of the FGFR, or against FGFR ligands or/and against downstream targets. The antibodies may be polyclonal antibodies or monoclonal antibodies, recombinant antibodies, e.g. single chain antibodies or fragments of such antibodies which contain at least one antigen-binding site, e.g. proteolytic antibody fragments such as Fab, Fab' or F(ab')2 fragments or recombinant antibody fragments such as scFv fragments. For therapeutic purposes, particularly for the treatment of humans, the administration of chimeric antibodies, humanized antibodies or human antibodies is especially preferred.

Furthermore, soluble FGFR4 receptors, e.g. receptor fragments without the membrane anchor domain, antagonistic FGFR4 ligand muteins, such as muteins of FGFs, peptides or low-molecular weight FGFR4 inhibitors may be used in the present disclosure. Examples of low-molecular weight inhibitors of FGFR4 are SU5402, SU4984 (Mohammadi et al., Science 1997 May 9;276(5314):955-601997), and PD17304 (Mohammadi et al., EMBO J., 17(20):5896-5904, 1998). In the experiments described herein, Fc decoy receptors, chimeric proteins which consist of an extracellular FGFR domain and and the Fc domain of a human immunoglobulin Gl, are described. These soluble FGF traps can be used to inhibit activation of FGFRs by FGFs.

### Inhibition via Small Molecules

Certain embodiments of the disclosure contemplate the inhibition of one or more FGFRs for the treatment of LVH through the use of a small molecule inhibitor. In certain embodiments such a small molecule inhibitor directly or indirectly inhibits the activation an FGFR. In other embodiments, the small molecule inhibitor inhibits or interferes with downstream signaling as a result of FGFR activation.

A non-limiting example of a small molecule inhibitor of FGFR4 which may be used in the treatment of LVH is PD173074, which has been reported by Mohammandi et al., 1998 and Grand et al., Leukemia 2004 May;18(5):962-6. 2004. PD173074 is a protein tyrosine kinase inhibitor based on a pyrido[2,3-d]pyrimidine core. This compound inhibits phosphorylation of FGFR3 and FGFR4. Other small molecule inhibitors of FGFR4 which may be used in the present disclosure for the treatment of LVH include SU6668 and SU5402. Other compounds which may be discovered and have the ability to inhibit generally the activation of fibroblast growth factor receptors, e.g., FGFR4, are also contemplated.

### Inhibition via Nucleic Acids

In certain embodiments, inhibition of one or more endocrine FGFs (FGF19, FGF21, and FGF23) or an FGFR is achieved through administration of nucleic acids which function to inhibit translation of mRNA encoding one or more FGFs and/or one or more FGFRs.

In specific embodiments wherein a nucleic acid may inhibit an FGFR (e.g., FGFR4), the nucleic acid may be, for example, shRNA. Taylor et al., (J Clin Invest. 2009 Nov;119(11):3395-407, 2009) describe a shRNA capable of inhibiting the translation of FGFR4 with the sequence of 5' - AGCTAAAAAGCCGTCAAGATGCTCAAAGACTCTCTTGAAGTCTTTGAGCATCTTG ACGGCGG-3' (SEQ ID NO:1). In other embodiments, the nucleic acid which may inhibit an FGFR (e.g., FGFR4) may be a siRNA, as described by Roidl et al., Clin Cancer Res. 2009 Mar 15;15(6):2058-66, 2009. One non-limiting example of a siRNA which inhibits FGFR4 is 5' - GGCUCUUCCGGCAAGUCAA-3' (SEQ ID NO:2).

### Inhibition via Antibodies

In certain embodiments, LVH is treated by inhibition of an FGFR (e.g., FGFR4) through the use of an antibody which blocks the ligand binding site of the FGFR (e.g., FGFR4) or otherwise partially or fully inhibits FGFR function. As such, an antibody may include any antibody including a fragment or derivative thereof that binds to the extracellular domain of an FGFR, e.g., of human FGFR4, and at least partially inhibits FGFR activity.

In an additional embodiment the present disclosure relates to a method of blocking FGFR function including contacting the antibody of the disclosure with cells or a tissue suspected of carrying FGFR on their/its surface under conditions, wherein the antibody is capable of blocking FGFR function. The contacting may be in vitro or in vivo.

Examples of monoclonal antibodies which are capable of blocking the function of FGFRs, e.g., FGFR4, may be found in U.S. Pub. No.: 20100169992.

Examples of the use of the administration of FGFR4 antibodies may be found in U.S. Pub. No.: 20100143386.

Examples of antibodies which are capable of binding to endocrine FGFs (FGF19, FGF21, and FGF23) may be found in U.S. Pub. No.: 20080241946, U.S. Pub. No.: 20090148461, and Desnoyers LR et al., Oncogene 2008, 27: 85-97.

The disclosure further relates to a method of treating a disease wherein the antibody of the disclosure is administered to a mammal and wherein said disease is correlated directly or indirectly with the abnormal level of expression or activity of FGFR4.

In certain embodiments, the antibody has at least one antigen binding site. Further, the antibody preferably comprises at least one heavy immunoglobulin chain and at least one light immunoglobulin chain. An immunoglobulin chain comprises a variable domain and optionally a constant domain. A variable domain may comprise complementary determining regions (CDRs), e.g. a CDR1, CDR2 and/or CDR3 region, and framework regions. The term "complementary determining region" (CDR) is well-defined in the art (see, for example, Harlow and Lane, "Antibodies, a laboratory manual", CSH Press, Cold Spring Harbor, 1988) and refers to the stretches of amino acids within the variable region of an antibody that primarily makes contact with the antigen.

The antibody may be any antibody of natural and/or synthetic origin, e.g. an antibody of mammalian origin. Preferably, the constant domain - if present - is a human constant domain. The variable domain is preferably a mammalian variable domain, e.g. a humanized or a human variable domain. More preferably, the antibody is a chimeric, humanized or human antibody.

The antibody of the disclosure may be of the IgA-, IgD-, IgE, IgG- or IgM- type, preferably of the IgG- or IgM-type including, but not limited to, the IgGI-, IgG2-, IgG3-, IlgG4-, IgMI- and 1gM2-type. In most preferred embodiments, the antibody is of the human IgGI-, IgG2- or IgG4- type.

The term antibody includes "fragments" or "derivatives", which have at least one antigen binding site of the antibody. Antibody fragments include Fab fragments, Fab 1 fragments F(ab')2 fragments as well as Fv fragments. Derivatives of the antibody include single chain antibodies, nanobodies, and diabodies. Derivatives of the antibody may also include scaffold proteins having an antibody-like binding activity that bind to FGFR4.

In an additionally preferred embodiment of the disclosure, the antibody may be a Fab-fragment, a F(ab2)'- fragment, a single-chain antibody, a chimeric antibody, a CDR-grafted antibody, a bivalent antibody-construct, a humanized antibody, a human, a synthetic antibody, or a chemically modified derivative thereof, a multispecific antibody, a diabody, a nanobody, a Fv- fragment, or another type of a recombinant antibody

### B. Inhibition of FGFs

In another embodiment of a method of treating or preventing a disease or condition associated with elevated FGF levels in a subject (e.g., human), the method includes administering to the subject a composition including a pharmaceutically acceptable carrier and an inhibitor of at least one FGF (e.g., one or more of FGF19, FGF21, and FGF23) in an amount effective for inhibiting expression or signaling of the at least one FGF in the subject. The disease or condition associated with elevated FGF levels can be, for example, one or more of cardiovascular disease (e.g., LVH, hypertension, and congestive heart failure), CKD, and diabetes. The method can further include administering to the subject an inhibitor of at least one FGFR in an amount effective for inhibiting expression or activation of the at least one FGFR and inhibiting signaling of at least one FGF in the subject. In this embodiment, the inhibitor of the at least one FGF and the inhibitor of expression or activation of the at least one FGFR can be administered to the subject at the same time, or administered to the subject at different times.

Certain embodiments of the disclosure relate to inhibition of one or more FGFs (e.g., FGF19, FGF21, and FGF23) in cardiac cells in a subject. In such embodiments, the inhibitor of an FGF may be a molecule that reduces the level of mRNA encoding the FGF polypeptide, a molecule that reduces the level of the FGF polypeptide, or a molecule that reduces a biological activity of the FGF polypeptide. Still further, the inhibitor is an antisense nucleic acid, a ribozyme, an antibody, a peptide or a peptidomometic. Compositions and methods which inhibit one or more FGFs (e.g., FGF19, FGF21, and FGF23) may be found in U.S. Pub. No. 20080241946.

### C. Prognostic or Diagnostic Applications

In one embodiment of a method of determining whether a subject (e.g., human, rodent, bovine, canine, ovine, etc.) having a disease or condition associated with elevated FGF levels is in need of inhibiting activation of FGFR signaling, the method includes: analyzing a database of subjects without the disease or condition and with normal blood levels of at least one FGF; obtaining a biological sample (e.g., blood, plasma, urine, saliva, etc.) from the subject having the disease or condition; determining if the level of the at least one FGF in the biological sample from the subject is elevated compared to a normal level based on the database; and correlating an increased level of the at least one FGF in the biological sample from the subject relative to the normal level based on the database with a need for inhibiting activation of FGFR signaling in the subject. The disease or condition can be one or more of: cardiovascular disease (e.g., LVH, hypertension, and congestive heart failure), diabetes, and CKD. Any suitable biological sample can be obtained and used for determining the level of the at least one FGF in the subject. In some embodiments, the method can be used with seemingly healthy individuals, in order to determine if they would benefit from or are in need of a therapeutic composition as described herein for inhibiting activation of FGFR signaling and/or expression or signaling of one or more FGFs.

A further aspect of the disclosure refers to the diagnosis of a subject in need of therapy to treat a disease or condition associated with elevated levels of at least one FGF, wherein a sample from a subject, which may suffer from or have risk factors for cardiovascular disease, diabetes, and/or CKD, is analyzed for gene or protein expression of one or more FGFs (e.g., FGF19, FGF21, and FGF23) or gene or protein expression of one or more FGFRs (e.g., FGFR4). The sample may, e.g., be a body fluid sample or a tissue sample, e.g. a biopsy. The FGFR or FGF expression may be determined on the nucleic acid level or on the protein level according to standard methods, e.g. using a gene array or immunochemical, e.g. ELISA or immunohistochemical methods. An overexpression of FGFR or one or more FGFs is determined by comparing the FGFR or FGF expression in the sample to be analyzed with the FGFR or FGF expression in control samples, e.g. samples from healthy subjects or with standard values. Normal ranges of endocrine FGFs levels in humans are known. For example, normal levels of FGF19 are typically 200-250 pg/ml (Mraz M et al., Physiol. Res. 60: 627-636, 2011). Normal levels of FGF21 are typically 200-250 pg/ml (Dostalova et al., J Clin Endocrinol Metab, September 2008, 93(9):3627-3632). Normal levels of FGF23 are typically 30 pg/ml (Wolf M, Kidney International, 2012).

The diagnostic composition of the disclosure is useful in the detection of an undesired expression, overexpression or hyperactivity of the mammalian FGFR in different cells, tissues or another suitable sample, comprising contacting a sample with an antibody of the disclosure, and detecting the presence of FGFR in the sample. Accordingly, the diagnostic composition of the disclosure may be used for assessing the onset or the disease status of cardiovascular disease.

Yet another aspect of the present disclosure is a screening method for identifying or/and characterizing a compound or agent suitable for reducing LVH, cardiovascular disease, CKD. diabetes, etc., which screening method includess determining, if the compound is an FGFR inhibitor.

In a particular embodiment, in the screening method of the present disclosure identifying the FGFR inhibitor includes the steps (a) contacting at least one compound with FGFR, and (b) determining FGRF activity in the presence of the compound.

The screening method of the present disclosure may include the use of isolated proteins, cell extracts, recombinant cells or/and transgenic non- human animals. In particular, the FGFR inhibitor may be identified in a molecular or/and cellular assay.

The recombinant cells or/and transgenic non-human animals preferably exhibit an altered FGFR expression, in particular an FGFR4 overexpression compared to a corresponding wild-type cell or animal.

In a screening method of the present disclosure, the FGFR inhibitor can be an inhibitor of positive NFAT and/or MAP kinase regulation by FGFR, or/and an inhibitor of FGFR-induced phenotypic changes (e.g. the development of hypertrophy). Thus, as indicated above, step (b) may include determination of the amount of FGFR, determination of NFAT or/and MAP kinase upregulation by FGFR or/and FGFR induced resistance to apoptosis induction.

The screening method of the present disclosure can be carried out in a high-throughput format for identifying novel compounds or classes of compounds. Further, the screening method of the present disclosure is suitable as a validation procedure for characterizing the pharmaceutical efficacy and/or the side effects of known compounds.

### D. Pharmaceutical Compositions and Routes of Administration

Yet another aspect of the present disclosure is a pharmaceutical composition or kit including a) an FGFR inhibitor as defined above, and (b) pharmaceutical formulation for delivery of the FGFR inhibitor.

Pharmaceutical compositions or kits suitable for use in the present disclosure include compositions or kits wherein the active ingredients are contained in an effective amount to achieve its intended purpose. A therapeutically effective dose refers to that amount of the compounds that results in amelioration of symptoms, prevention of the onset of symptoms or a prolongation of survival in a patient suffering from a cardiovascular disease such as hypertension, congestive heart failure or LVH.

Toxicity and therapeutic efficacy of the FGFR inhibitor and the agent for the prevention, alleviation or/and treatment of CKD, diabetes, or a cardiac disease such as hypertension, congestive heart failure or LVH, can be determined by standard pharmaceutical procedures in cell cultures or experimental animals, e.g. for determining the LD₅₀ (the dose lethal to 50% of the population) and the ED₅₀ (the dose therapeutically effective in 50% of the population). For any compound used in the present disclosure, the therapeutically effective dose can be estimated initially from cell culture assays. For example, a dose can be formulated in animal models to achieve a circulating concentration range that includes the IC₅₀ as determined in cell culture (i.e. the concentration of the test compound which achieves a half- maximal inhibition of the growth-factor receptor activity). Such information can be used to more accurately determine useful doses in humans. The dose ratio between toxic and therapeutic effects is the therapeutic index and it can be expressed as the ratio between LD₅₀ and ED₅₀. Compounds which exhibit high therapeutic indices are preferred. The exact formulation, route of administration and dosage can be chosen by the individual or physician in view of the patient's condition (see e.g. Fingl et al., 1975, in "The Pharmacological Basis of Therapeutics", Ch. 1 , p. 1). Dosage amount and interval may be adjusted individually to provide plasma levels of the active moiety which are sufficient to maintain the receptor modulating effects, or minimal effective concentration (MEC). The MEC will vary for each compound but can be estimated from in vitro data, e.g. the concentration necessary to achieve a 50-90% inhibition of the receptor using the assays described herein. Compounds should be administered using a regimen which maintains plasma levels above the MEC for 10-90% of the time, preferably between 30-90% and most preferably between 50-90%. Dosages necessary to achieve the MEC will depend on individual characteristics and route administration. In cases of local administration or selective uptake, the effective local concentration of the drug may not be related to plasma concentration.

The actual amount of the pharmaceutical composition or kit administered is dependent on the subject being treated, on the subject's weight, the severity of the affliction, the administration route and the judgment of the prescribing physician. For antibodies or therapeutically active nucleic acid molecules, and other compounds e.g. a daily dosage of 0,001 to 100 mg/kg, particularly 0.01 to 10 mg/kg per day is suitable. In one embodiment, a dosage of 25 mg/kg is administered twice a week (injected i.p.).

Suitable routes of administration include, for example, oral, rectal, transmucosal or intestinal administration; parenteral delivery, including intramuscular, subcutaneous, intramedullary injections, as well as intrathecal, direct intraventricular, intravenous, intraperitoneal, intranasal or intraocular injections.

The term "composition" as employed herein comprises at least one compound of the disclosure. Preferably, such a composition is a pharmaceutical or a diagnostic composition.

It is preferred that said pharmaceutical composition includes a pharmaceutically acceptable carrier and/or diluent. The herein disclosed pharmaceutical composition may be useful for the treatment of disorders associated with, accompanied by or caused by FGFR expression, FGFR activation, FGF (e.g., FGF19, FGF21, FGF23) expression or FGF (e.g., FGF19, FGF21, FGF23) overexpression.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The compositions of the disclosure may also be administered directly to the target site, e.g., by biolistic delivery to an external or internal target site, like the brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 µg and 100 mg/kg body weight per dose; however, doses below or above this exemplary range are envisioned, especially considering the aforementioned factors. If the regimen is a continuous infusion, it should also be in the range of 1 pg to 100 mg per kilogram of body weight per minute.

Progress can be monitored by periodic assessment. The compositions of the disclosure may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the disclosure may comprise further agents depending on the intended use of the pharmaceutical composition.

In embodiments wherein more than one inhibitor is contemplated to directly and/or indirectly inhibit at least one FGFR (e.g., FGFR1, FGFR2, FGFR3, FGFR4) and/or at least one endocrine FGF (FGF19, FGF21, FGF23), the inhibitors may be provided in one pharmaceutical composition (single dose form) or may be provided in distinct compositions (separate dose form), or in a single composition. In particular, the distinct compositions of the pharmaceutical composition of the disclosure may be administered by the same route or by different routes.

By their citation of various references in this document, Applicants do not admit any particular reference is "prior art" to their disclosure.

### EXAMPLES

The following examples are included to demonstrate preferred embodiments of the disclosure. It should be appreciated by those of skill in the art that the techniques disclosed in the examples which follow represent techniques discovered by the inventors to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit or scope of the disclosure. The following Examples are offered by way of illustration and not by way of limitation.

### Example 1: Fibroblast Growth Factor 23 Induces Left Ventricular Hypertrophy

### Materials and Methods

### Growth Factors

We used recombinant mouse FGF23 (6His-tagged Tyr25-Va1251 (Arg179Gln)), FGF2 (Alall-Ser154), and FGF4 (Ala30-Leu202) proteins (R&D Systems). We chose the mutant form of FGF23 because it is resistant to furin protease-mediated degradation, which prolongs the protein's half-life. We confirmed in vitro biological activity of FGF23 by detecting increased levels of phospho-ERK and Egr-1 in response to treatment with FGF23 in HEK293 cells that transiently express FLAG-tagged klotho, as has been done previously (Kurosu et al., 2006). We confirmed in vivo biological activity of FGF23 by demonstrating significantly decreased serum phosphate levels over 4 hours after a single intravenous injection of 40 µg/kg of purified protein dissolved in 0.5 ml of PBS in 3 male Sprague-Dawley rats (Charles River), as has been done previously (Shimada et al., J. Clin. Endocrinol. Metab., 95(2):578-585, 20102001).

### Primers

The Table 1 lists the intron-spanning oligonucleotides (shown in 5' to 3' orientation) which were used as primers in RT-PCR and nested PCR analyses.

| Table 1 | | |
|---|---|---|
| Gene | Orientation | Primer Sequence (5' to 3') |
| a-MHC | Forward | CTTCACAGCAGAGGAGAAGG (SEQ ID NO:3) |
| | Reverse | ACACCTGCTGTACACTCTGC (SEQ ID NO:4) |
| f3-MHC | Forward | CTCCAGAAGAGAAGAACTCC (SEQ ID NO:5) |
| | Reverse | CCACCTGCTGGACATTCTGC (SEQ ID NO:6) |
| ANP | Forward | AGCGAGCAGACCGATGAAGC (SEQ ID NO:7) |
| | Reverse | AGCAGCTTGACCTTCGCAGG (SEQ ID NO:8) |
| BNP | Forward | CCAGATGATTCTGCTCCTGC (SEQ ID NO:9) |
| | Reverse | TGAACTATGTGCCATCTTGG (SEQ ID NO:10) |
| MCAD | Forward | CGGTGCTCTGACACCAGAG (SEQ ID NO:11) |
| | Reverse | AGAGGCAAAGTACGTGTTCC (SEQ ID NO:12) |
| FGFR1 (b, c) | Forward | TGCCAGCTGCCAAGACGGTG (SEQ ID NO:13) |
| | Reverse | AAGGATGGGCCGGTGAGGGG (SEQ ID NO:14) |
| FGFR2 (b, c) | Forward | GCTCCATGCTGTCCCTGCCG (SEQ ID NO:15) |
| | Reverse | TCCCCGAGTGCTTCAGGACC (SEQ ID NO:16) |
| FGFR3 (b, c) | Forward | AGTGTTCTGCGTGGCGGTCG (SEQ ID NO:17) |
| | Reverse | GCACAGCACACGCCGGGTTA (SEQ ID NO:18) |
| FGFR4 | Forward | GGCTATGCTGTGGCCGCACT (SEQ ID NO:19) |
| | Reverse | GGTCTGAGGGCACCACGCTC (SEQ ID NO:20) |
| aKlotho | Forward (Outside) | GACTTTCTGAGTCAGGACAAGG (SEQ ID NO:21) |
| | Reverse (Outside) | GTTACCCAGAGGCAAGATCAGG (SEQ ID NO:22) |
| aKlotho | Forward (Nested) | GTCTTCGGCCTTGTTCTACC (SEQ ID NO:23) |
| | Reverse (Nested) | CGAAGTAAGGTTATCTGAGG (SEQ ID NO:24) |
| GAPDH | Forward | TATGTCGTGGAGTCTACTGG (SEQ ID NO:25) |
| | Reverse | AGTGATGGCATGGACTGTGG (SEQ ID NO:26) |

### Isolation and culture of NRVM

NRVM were isolated using a standard isolation system (Worthington Biochemical Corporation) (Toraason et al., Toxicology, 56(1):107-117, 1989). Hearts from 1-2 day old Sprague Dawley rats (Charles River) were harvested, minced in calcium- and magnesium-free Hank's Balanced Salt Solution (HBSS), and the tissue digested with 50 µg/ml trypsin at 4°C for 20-24 hours. Soybean trypsin inhibitor in HBSS was added and the tissue was further digested with collagenase (in Leibovitz L-15 medium) under slow rotation (15 rpm) at 37°C for 45 minutes. Cells were released by gently triturating the suspension 20 times with a standard 10 ml plastic serological pipette and filtering it twice through a cell strainer (70 lam, BD Falcon). Cells were incubated at room temperature for 20 minutes and spun at 100 g for 5 minutes. The cell pellet was resuspended in plating medium [Dulbecco's Modified Eagle Medium (DMEM; Cellgro) with 17% Media 199 (Invitrogen), 15% fetal bovine serum (FBS; Invitrogen) and 1% penicillin/streptomycin solution (P/S; Invitrogen)]. Cells were counted using a hemocytometer.

Cells were plated on glass and plastic surfaces which were coated with laminin (Invitrogen; 10 µg/ml in PBS) at room temperature for 1 hour prior to plating. For immunofluorescence analysis, 1 x 10⁶ cells were seeded per well on glass coverslips in 6-well plates. For protein and RNA isolation, 2 x 10⁶ cells were seeded in 6 cm-culture dishes. Cells were left undisturbed in plating medium at 37°C for 72 hours and then cultured in maintenance medium (DMEM with 20% Media 199, 1% insulin-transferrin-sodium selenite solution (ITS; Sigma-Aldrich) and 1% P/S) in the presence of 100 µM 5-bromo-2'-deoxyuridine (BrdU; Sigam-Aldrich) to eliminate proliferating non-myocytes resulting in a relatively pure population of isolated cardiac myocytes (Bishopric et al., 1987). After 4 days, isolated cardiac myocytes were cultured in BrdU-containing maintenance medium in the presence of recombinant murine FGF23 (26.1 kDa, 251 amino acids; R&D Systems) or FGF2 (16.2 kDa, 144 amino acids; R&D Systems) for 48 hours.

### RNA isolation and quantification

RNA was purified from isolated NRVM using the RNeasy kit (Qiagen), and from mouse cardiac and renal tissue using 1 ml per 100 mg tissue TRIzol (Invitrogen), following the manufacturers' protocol. Prior to RT-PCR, total RNA samples were digested with DNase I (Roche) and RNA was transcribed into cDNA using Superscript II (Invitrogen). For RT-PCR, 100 ng cDNA, AmpliTaq Gold DNA polymerase (Applied Biosystems) and sequence specific primers were used. 30 cycles (95°C, 5 minutes - 53°C, 30 seconds - 72°C, 30 seconds) were run on a GeneAmp PCR System 9700 (Applied Biosystems) and reactions were analyzed on a 2% agarose gel. Ethidium bromide signals were captured with a Bio-Rad Gel Doc XR system and quantified by densitometry using ImageJ software (NIH). Values were normalized to GAPDH signals and cDNAs from three independent experiments were analyzed. For nested PCR, 100 ng cDNA were used in the first run (15 cycles) with sequence specific outside primers. Five percent of the reaction was used as template for the second run (25 cycles) with sequence specific inside primers, and the total reaction was analyzed by agarose gel electrophoresis.

### Protein isolation

NRVM were scraped from a 6 cm-plate using 400 pi CHAPS extraction buffer [(20 mM Tris-HCl pH7.5, 500 mM NaCl, 0.5% CHAPS, protease inhibitor cocktail (Roche), protein phosphatase inhibitors (Sigma-Aldrich)] and incubated on ice for 15 minutes. The cell lysate was centrifuged at 14,000 rpm and 4°C for 15 minutes and the supernatant boiled in sample buffer and analyzed by SDS-PAGE and immunoblotting as described before (Faul et al., Cell Biol., 169(3):415-424, 2005; Faul et al., Mol. Cell Biol., 27(23):8215-8227, 2007). For protein extraction from mouse hearts, tissue was isolated, minced and homogenized in CHAPS extraction buffer at 1:10 (w:v) and protein lysates were prepared as described above. Antibodies to sarcomeric α-actinin (EA-53; Sigma-Aldrich; 1:1000), total (Cell Signaling; 1:1000) and phospho-ERK (Cell Signaling; 1:1000), total and phospho-NFAT-cl (Santa Cruz Biotechnology; 1:200), total and phospho-Akt (Cell Signaling; 1:1000 and 1:2000), and GAPDH (Abcam; 1:10,000) were used as primary antibodies and horseradish peroxidase conjugated goat-anti-mouse and goat-anti-rabbit (Promega; 1:20,000) were secondary antibodies.

### Immunocytochemistry and morphometry

We analyzed isolated NRVM on laminin-coated glass coverslips by immunocytochemsitry according to our established protocols (Faul et al., Cell Biol., 169(3):415-424, 2005; Faul et al.,.Cell Biol., 27(23):8215-8227, 2007). The mouse monoclonal antibody against sarcomeric α-actinin (EA-53; Sigam-Aldrich) was used at 1:1000 and Cy3-conjugated goat-anti mouse (Jackson Immuno Research) as secondary antibody at 1:750. To visualize nuclei, fixed cells were incubated with 4',6-diamidino-2-phenylindole (DAPI; 400 ng/ml in PBS) for 5 minutes. Immunofluorescence images were taken on a Leica TCS-SP5 confocal microscope with a 63x oil objective with a 1.6x zoom. Leica AF6000 fluorescence software was used to measure the cell surface area based on a-actinin-positive staining.

To assess the signaling pathways involved in FGF23- and FGF2-mediated hypertrophy, we analyzed the morphometry of plated cells that were pre-treated with specific inhibitors for 60 minutes prior to the addition of the FGFs at varying concentrations. Table 2 lists the inhibitors used and their concentrations.

| **Table 2** | | |
|---|---|---|
| **Inhibitor (Company)** | **Enzyme or receptor inhibited** | **Concentration** |
| | | |
| PD173074 (Sigma-Aldrich) | pan-FGFR | 10 nM |
| U0126 (Cell Signaling) | ERK1/2 | 10 µM |
| PD98059 (Cell Signaling) | ERK1 | 20 µM |
| Wortmannin (Cell Signaling) | PI3K | 500 nM |
| A6730 (Sigma-Aldrich) | Akt1/2 | 200 nM |
| U73122 (Sigma-Aldrich) | Phospholipase C and A₂ | 10 µM |
| Cyclosporine A (Sigma-Aldrich) | Calcineurin | 0.83 µM |

### Intra-myocardial injections in mice

Twelve-week old adult C57BL/6 mice (Jackson Laboratories) underwent cardiac surgery using a standard operative procedure that has been described previously for intra-myocardial implantation of stem cells (Amado et al., Proc. Natl. Acad. Sci. U. S. A., 102(32):11474-11479, 2005). All procedures were performed by a single investigator who was blinded to the specific treatment group. All protocols and experimental procedures were approved by the Institutional Animal Care and Use Committee at the University of Miami Miller School of Medicine. Briefly, mice were anesthetized with isoflurane and an incision was made between the 3rd and 4th rib to reveal a cardiac window. Three injections of purified recombinant murine FGF23 in PBS or PBS alone were delivered into the free wall of the left ventricle via a 30-gauge needle attached to a standing Hamilton syringe. At each of the three sites, 10 µl were injected for a total of 30 µl containing a total of 7.5 ng FGF23. Cardiac function was evaluated by Vevo 770 imaging system (Visual Sonics, Toronto, Canada) at baseline and 2 weeks after surgery. Long and short axis 2D parasternal views and short axis M mode views were recorded under general anesthesia with isoflurane inhalation delivered through a nose mask. The heart rate was maintained above 400 bpm with controlled body temperature set to 37°C. At 7- and 14-days post-injection, animals were sacrificed, hearts were isolated, perfused ex-vivo (6 ml of 10% Formalin with 4 ml of 20 mM KC1), stored overnight in 10% formalin for fixation, and then serially sectioned (7 i.tm slices) and stained with hematoxylin and eosin. Age-matched untreated animals were sacrificed at 14 weeks of life. Age-matched untreated animals were sacrificed at 14 weeks of life.

### Intravenous injections in mice

Twelve-week old adult C57BL/6 mice underwent tail vein injections using standard protocols as previously described for the systemic delivery of plasmid DNA (Faul et al., 2008). Briefly, mice were placed in a restrainer and 40 µg/kg of FGF23 dissolved in 200 pi of PBS were injected into the tail vein of 5 mice twice daily with eight hours in between injections for 5 consecutive days. Four mice underwent the same injection schedule using 200 pi of PBS alone as negative control. On the morning of the 6th day, 16 hours after the final tail vein injections, animals were sacrificed and the hearts were isolated and prepared using the same protocol as described above.

### Morphology and molecular analysis of murine hearts

Short-axis cardiac sections were used to quantify myocardial thickness by measuring the distance from the inner to the outer myocardial zone at the mid-chamber zone. Mean left ventricular free wall thickness was calculated from seven measurements of wall thickness taken at 0, 30, 60, 90, 120, 150 and 180 degrees along the hemi circle of the short axis of the free wall; the mean of 7 comparable measurements along the hemi circle of the short axis of the interventricular septum were used to calculate mean septal thickness. Since klothro-ablated kl/kl mice display severe growth retardation with small hearts (Kuro-o et al., Nature, 390(6655):45-51, 1997), we also measured relative wall thickness calculated as two times the anterior wall thickness divided by left ventricular internal diameter. Total RNA was isolated from flash frozen hearts using TRIzol (Invitrogen) and RT-PCR analyses were performed as described above.

### Immunocytochemistry and morphometry

We measured cross-sectional surface area of cardiac myocytes in paraffin-embedded short-axis sections of the heart. Briefly, 7 µm thick sections were deparaffinized according to our established protocols. To specifically label cardiac myocytes, we used mouse monoclonal MF20 antibody against sarcomeric myosin at 1:200 and Cy5-conjugated donkey-anti mouse (Invitrogen) as secondary antibody at 1:400. To visualize nuclei, fixed tissue was incubated with 4',6-diamidino-2-phenylindole (DAPI; 400 ng/ml in PBS) for 5 minutes. To visualize cellular borders, fixed tissue was incubated with wheat germ agglutinin (WGA) conjugated to Alexa Fluor 555 (Invitrogen) at 1 mg/ml in PBS containing 10 mM sodium azide. Immunofluorescence images were taken on a Leica TCS-SP5 confocal microscope with a 63x oil objective. Leica AF6000 fluorescence software was used to quantify cross-sectional cell surface area of 25 cells per field at four fields along the mid-chamber free wall based on WGA-positive staining.

### Serology

Blood was collected at sacrifice via cardiac puncture and was centrifuged at 4°C and 14,000 rpm for 15 minutes. Plasma supernatants were collected, stored at -80°C, and subsequently analyzed in batches for FGF23 (C-terminal mouse assay, Immutopics), PTH (1-84 assay, Alpco Immunoassays), and phosphate, calcium, BUN and creatinine (Ortho Vitros 250 Chemistry Analyzer).

### Statistical analysis of in vitro and animal data

Data are presented as means ± SEM. Analysis of variance and t-tests were used for statistical inference with two-tailed P values < 0.05 considered significant.

### Results

Plasma FGF23 is increased in CKD patients and independently associates with LVH. We measured FGF23 levels in baseline plasma samples from 3044 participants who underwent echocardiography 1 year later in the prospective Chronic Renal Insufficiency Cohort study. The population consisted of 46% women, 42% black, and 13% Hispanic patients, with a median age of 60 years, median blood pressure of 125/71 mmHg, and a median estimated glomerular filtration rate of 42 ml/min/1.73m2. The median FGF23 of 142 RU/ml was more than three-fold greater than in previous studies of predominantly non-CKD patients (Figure 1A) (36). Mean (± standard error) ejection fraction was 54 ± 0.2%, left ventricular mass indexed to height2.7 (LVMI) was 52 ± 0.3 g m-2.7 (normal range: <50 in men; <47 in women), and LVH was present in 53% of patients.

When examined according to quartiles of FGF23, ejection fraction was modestly reduced in the highest versus the lower FGF23 quartiles (Figure 1B), while LVMI increased monotonically with increasing FGF23 quartiles (Figure 1C). Each unit increase in log-transformed FGF23 was associated with a 5.1 g m-2.7 increase in LVMI (95% confidence interval [CI] 4.4, 5.7; P < 0.001). The prevalence of normal left ventricular geometry decreased with increasing quartiles of FGF23 while the prevalence of eccentric and concentric LVH increased significantly (Figure 1D). Each unit increase in logFGF23 was associated with a 2.5-fold increased relative risk (RR) of eccentric hypertrophy and concentric hypertrophy (95%CI 2.1, 2.9; P < 0.001 for each) compared to normal ventricular geometry.

In multivariable analyses that adjusted for age, sex, race, ethnicity, weight, smoking, systolic blood pressure, history of cardiovascular disease, diabetes, total cholesterol, estimated glomerular filtration rate, hemoglobin, albuminuria, parathyroid hormone (PTH), and phosphate, high FGF23 remained an independent predictor of increased LVMI (1.5 g m-2.7 increase per unit increase in logFGF23, 95%CI 0.9, 2.2; P < 0.001), and increased risk of eccentric (RR 1.5 per unit increase in logFGF23; 95%CI 1.2, 1.9; P = 0.001) and concentric hypertrophy (RR 1.6 per unit increase in logFGF23; 95%CI 1.3, 1.9; P < 0.001). In addition to elevated FGF23, older age, black race, Hispanic ethnicity, prior history of cardiovascular disease, higher blood pressure and body weight, anemia, and higher levels of proteinuria, parathyroid hormone and phosphate also remained associated with LVH in the full multivariable model. The independent association between higher FGF23 and LVH was consistent across genders, races, ethnicities, CKD stages, and patients with and without diabetes, hypertension, or a history of prior cardiovascular events, and across patients who were or were not treated with anti-hypertensive agents (data not shown). Furthermore, the results were unchanged when we adjusted for levels of 25-hydroxyvitamin D and 1,25-dihydroxyvitamin D in the subset of 1043 patients in whom these were available (data not shown). These data indicate a robust association between FGF23 and LVH across broad groups of patients with CKD.

FGF23 induces hypertrophy and activates pro-hypertrophic gene programs in isolated neonatal cardiac myocytes. We tested the hypothesis that the independent association between high FGF23 levels and increased risk of LVH in CKD patients is mediated by a direct effect of FGF23 on cardiac myocytes. We compared the response of isolated neonatal rat ventricular cardiac myocytes (NRVM) to 48 hours of incubation with FGF23 versus FGF2 at concentrations that have been previously reported in experiments that demonstrated direct hypertrophic effects of FGF2 (Parker et al., J. Clin. Invest., 85(2):507514, 1990; Kardami et al Cardiovasc. Res., 63(3):458-466, 2004). Since the development of LVH involves an increase in the contractile machinery and thus sarcomere number, we analyzed the expression of a-actinin, which anchors actin filaments to the sarcomeric Z-disc (Kostin et al., Heart Fail. Rev., 5(3):271-280, 2000). Immunocytochemical and morphometric analyses revealed a significant increase in cell surface area in response to FGF23 and FGF2 (Figures 2A,B), while immunoblotting showed an increase in α-actinin protein levels (Figure 2C), indicative of increased sarcomeric content. In contrast to FGF23 and FGF2, we observed no change in size of cardiac myocytes in response to FGF4, which excludes a non-specific FGF effect.

Since these findings suggest induction of hypertrophic growth of NRVM, we used RT-PCR analysis to determine the expression levels of genes that are established markers of pathological cardiac hypertrophy (Figure 2D). Expression of adult a-myosin heavy chain (a-MHC) decreased while expression of fetal f3-myosin heavy chain (f3-MHC) increased after FGF23 and FGF2 treatment. This switch from adult to fetal MHC isoforms indicates reactivation of fetal gene programs that are associated with cardiac hypertrophy (Morkin, E Microsc. Res. Tech., 50(6):522-531, 2000; Izumo et al., J. Clin. Invest., 79(3):970-977, 1987; Lompre et al., Nature, 282(5734):105-107, 1979; Molkentin et al., Cell, 93(2):215-228, 1998). In addition, FGF23 and FGF2 increased expression of atrial natriuretic peptide (ANP) and brain natriuretic peptide (BNP), which are established markers of LVH (Molkentin et al., Cell, 93(2):215-228, 1998, Komuro and Yazaki, Annu. Rev. Physiol., 55:55-75, 1993). FGF23 and FGF2 treatment also decreased expression of medium chain acyl-CoA dehydrogenase (MCAD), an enzyme that regulates fatty acid oxidation. Cardiac myocytes that undergo hypertrophy shift their primary energy source from fatty acids to carbohydrates (Barger and Kelly, Am. J. Med. Sci., 318(1):36-42, 1999), which is marked by reduced expression of MCAD (Rimbaud et al., J. Mol. Cell Cardiol., 46(6):952959, 2009). Collectively, these data demonstrate that FGF23 and FGF2 exert similar direct hypertrophic effects on isolated NRVM.

FGF23-mediated hypertrophy is FGF receptor-dependent but klotho-independent. Klotho is the primary co-receptor for FGF23 in the kidney and parathyroid glands but is not expressed in cardiac myocytes (Urakawa et al., .Nature, 444(7120):770774,. 2004; Kuro-o et al., Nature, 390(6655):45-51, 1997). To confirm that the hypertrophic response to FGF23 was independent of klotho, we analyzed klotho expression by nested RT-PCR using two sets of klotho-specific primers in two consecutive PCRs in order to detect even the smallest amounts of cDNA. We detected klotho mRNA in mouse brain and kidney, which are known to express klotho (Urakawa et al., .Nature, 444(7120):770-774,. 2004), but not in isolated NRVM or total heart preparations (Figure 3A).

The biological functions of FGFs are mediated by FGFR1-4, which are members of the receptor tyrosine kinase family (Jaye et al., Biochim. Biophys. Acta., 1135(2):185-199, 1992). Since FGF23 can bind the four FGFR isoforms with varying affinity (Zhang et al., J. Biol. Chem., 281(23):15694-15700, 2006; Yu et al., Endocrinology, 146(11):4647-4656, 2005), we analyzed and detected expression of all FGFR isoforms in liver, isolated NRVM and total murine heart (Figure 3B). To determine, if the pro-hypertrophic effect of FGF23 on cardiac myocytes is dependent on FGFR, we co-treated isolated NRVM with FGF23 or FGF2 and the pan-FGFR inhibitor PD173074 (Mohammadi et al., 1998). The presence of PD173074 completely blocked FGF23- and FGF2-induced increases in cell surface area regardless of FGF concentration (Figure 3C). These findings demonstrate that FGF23 induces hypertrophy of isolated NRVM via FGFR activation, but through a klotho-independent pathway.

Activation of isolated NRVM by FGF23 and FGF2 utilizes distinct signaling pathways. FGFR signaling involves activation of the MAPK cascade in which the extracellular signal-regulated kinase (ERK) plays a central role (Eswarakumar et al., Cytokine Growth Factor Rev., 16(2):139-149, 2005; Katz et al., Biochim. Biophys. Acta., 1773(8):1161-1176, 2007). Once phosphorylated in cardiac myocytes, ERK activates a wide array of targets (Muslin AJ., Clin. Sci. (Lond)., 115(7):203-218, 2008; Sugden and Clerk, Circ. Res., 83(4):345-352, 1998; Bueno et al., EMBO J., 19(23):6341-6350, 2000), including the early growth response (Egr)-1 transcription factor (Buitrago et al., Nat. Med., 11(8):837-844, 2005), that alter gene expression and contribute to the induction of hypertrophy. Since FGF2 employs ERK as a downstream mediator of its hypertrophic effect in cardiac myocytes (Bogoyevitch et al., J. Biol, Chem., 269(2):1110-1119, 1994), and FGF23 signals via ERK activation in its primary target cells (Urakawa et al., Nature, 444(7120):770-774, 2004; Kurosu et al., J. Biol. Chem., 281(10):6120-6123, 2006), we tested whether FGF23 also activates ERK in isolated NRVM. As expected, FGF2 stimulated a robust increase in phosphorylated ERK, the activated kinase form, while total ERK levels did not change (Figure 3D). In addition, FGF2 stimulated an increase in Egr-1 levels (Figure 3D). In contrast, we detected only a modest increase in phosphorylated ERK and Egr-1 levels in isolated NRVM treated with FGF23 (Figure 3D). Consistent with these data, when we co-treated isolated NRVM with the ERK inhibitors U0126 or PD98059, FGF2-induced hypertrophy was completely prevented, whereas FGF23-induced hypertrophy was only partially diminished (Figure 3C). These data suggest that whereas the MAPK cascade is a dominant signaling pathway of FGF2-mediated cardiac hypertrophy, FGF23 appears to utilize additional signaling pathways.

To identify alternative signaling cascades underlying FGF23-induced pathological hypertrophy of cardiac myocytes, we studied phospholipase Cy (PLCy), which is another downstream mediator of FGFR signaling (Eswarakumar et al., J. Am. Soc. Echocardiogr., 18(12):1440-1463, 2005). The presence of U73122, which inhibits PLCy, attenuated FGF23-mediated hypertrophy, but had no effect on FGF2-treated cells (Figure 3C). An important downstream target of PLCy is the calcineurin-NFAT cascade (Crabtree and Olson, Cell, 109 Suppl:S67-79 2002), which is a central signaling pathway in the pathogenesis of LVH (Molkentin et al., Cell, 93(2):215-228, 1998; Wilkins and Molkentin, Biochem. Biophys. Res. Commun., 322(4):1178-1191, 2004). The presence of the calcineurin inhibitor cyclosporine A attenuated FGF23-mediated hypertrophy to a virtually identical extent as U73122 but had no effect on FGF2-treated cells (Figure 3C). In addition, FGF23 stimulated a modest decrease in phosphorylated NFAT after 60 minutes, suggestive of enhanced calcineurin activity, whereas FGF2 had no effect (Figure 3E). These data indicate that the PLCy-calcineurin-NFAT axis is a dominant signaling pathway of FGF23-mediated cardiac hypertrophy.

Finally, we analyzed the PI3K-Akt signaling axis, which has been implicated in the development of physiological rather than pathological hypertrophy of cardiac myocytes (Heineke and Molkentin, Nat. Rev. Mol. Cell. Biol., 7(8):589-600, 2006; Shioi et al., EMBO J., 19(11):2537-2548, 2000). The presence of the PI3K inhibitor Wortmannin or the Akt inhibitor A6730 partially attenuated FGF2-induced hypertrophy but did not significantly alter hypertrophic growth of FGF23-treated cells (Figure 3C). Consistent with this finding, FGF2 alone stimulated a modest increase in phosphorylated Akt, the activated kinase form (Figure 3F). Based on these data, we conclude that activation of the PI3K-Akt signaling cascade is not centrally involved in FGF23-mediated cardiac hypertrophy.

Direct myocardial delivery of FGF23 induces left ventricular hypertrophy in mice. To determine whether the direct pro-hypertrophic effect of FGF23 on isolated NRVM could be replicated in vivo, we injected FGF23 directly into the left ventricular myocardium of mice. Using a standard procedure for implanting stem cells into murine hearts in vivo (Amado et al., Proc. Natl. Acad. Sci. U. S. A., 102(32):11474-11479, 2005), we delivered 7.5 ng FGF23 protein dissolved in 30 µl PBS, or 30 µl of PBS alone, via three separate injection sites (10 µl each) into the anterior wall of the left ventricle of adult wild type mice. We opted to inject FGF23 directly into the myocardium in order to minimize the possibility of confounding by changes in circulating concentrations of other mineral metabolites that are regulated by FGF23 and could impact the development of LVH. Serologic testing confirmed minimal systemic absorption of FGF23, as there were no significant differences in levels of FGF23, phosphate, calcium, PTH, BUN, or creatinine between the FGF23- and vehicle-injected animals at days 7 and 14 after injection (data not shown).

The ratio of heart weight to tibial length, an established measure of cardiac hypertrophy (Antos et al., Proc. Natl. Acad. Sci. U. S. A., 99(2):907-912, 2002), was significantly increased at day 14 after FGF23 compared with vehicle injection (Figure 4A). Compared with vehicle, FGF23 induced a significant increase in thickness of the left ventricular free wall at day 7, which increased further by day 14 (Figure 4B). Thickness of the interventricular septum increased significantly by day 14 after FGF23 injection compared to vehicle, but the thickness of the ventricular free wall, where FGF23 was injected, was significantly greater than the septum at day 14 (Figure 4B). This suggests a gradient of effect related to the proximity to the injection sites where the concentration of FGF23 was likely highest. Representative gross pathology sections demonstrate FGF23-mediated LVH with thickened walls and diminished internal chamber size (Figure 4C). These results were consistent with echocardiography data that revealed no change in ejection fraction over time in FGF23 or vehicle-injected mice but significantly decreased left ventricular internal diameter in diastole and increased relative wall thickness (ratio of wall thickness to chamber diameter) by day 14 in the mice injected with FGF23 (Figure 4D). Microscopic analysis of cardiac myocytes stained with a fluorochrome-labeled wheat germ agglutinin (WGA) revealed a significant increase in cross-sectional surface area indicating hypertrophic myocyte growth (Figure 4C). Consistent with the in vitro results, cardiac injections of FGF23 caused a decrease in a-MHC and MCAD mRNA levels, and an increase in expression of f3-MHC, ANP and BNP (data not shown). In addition, klotho was not detected in the mouse hearts at baseline, and it was not induced at 7 or 14 days after injection. From these data we conclude that FGF23 induces LVH directly in vivo, and independently of klotho.

High systemic levels of FGF23 results in left ventricular hypertrophy in mice. The local concentration of FGF23 in the myocardium following intra-cardiac injection was likely significantly higher than in physiologic and pathological states. Therefore, we administered FGF23 intravenously to determine if systemically elevated FGF23 levels could also induce LVH. We injected 40 µg/kg of recombinant FGF23 dissolved in 200 µl of PBS into the tail vein of adult wild type mice twice daily for 5 days with eight hours between daily doses. The dose of FGF23 was chosen based on published reports in which a similar dose stimulated ERK phosphorylation and increased Egr-1 expression (Urakawa et al., Nature, 444(7120):770-774, 2004). The frequency of injections was chosen based on a pharmacokinetic study of adult wild type rats in which we injected a single dose of 40 µg/kg of FGF23. The half-life of circulating FGF23 was 30 minutes and plasma levels remained elevated above baseline at four hours post injection. Thus, we estimated that two daily injections of 40 µg/kg would provide elevated FGF23 levels for at least 8 hours per day in mice. Control animals underwent the same injection schedule using 200 µl of PBS alone. On the morning of day 6, blood was collected for assay of FGF23, mice were sacrificed and the hearts examined.

FGF23 levels measured at the time of sacrifice (16 hours after the final injection) were significantly increased compared with PBS-injected mice (Figure 5A). This indicates that FGF23 levels were likely persistently elevated throughout the study period. Compared with vehicle, FGF23-injected mice developed significantly increased cardiac weight/tibial length (Figure 5B), increased left ventricular wall thickness (Figure 5C, D), increased cross-sectional surface area of individual cardiac myocytes (Figure 5C, E), increased expression of I3-MHC, ANP and BNP, and decreased expression of a-MHC and MCAD mRNA levels (Figure 5F). These results demonstrate that intravenous injection of FGF23 yielded a cardiac phenotype that is similar to the phenotype induced by direct intra-myocardial injection of FGF23.

Left ventricular hypertrophy develops in a genetic mouse model of elevated FGF23. Finally, to determine if LVH develops in a "physiological" model of elevated FGF23 levels, we analyzed homozygous klotho-ablated (kl/kl) and klotho heterozygous (K1/+) mice. The kidneys of kl/kl mice are resistant to the phosphaturic effect of FGF23, which causes significant increases in circulating FGF23 levels. As reported previously [ref Kuro], kl/kl (9.7 ± 0.2 g) were significantly smaller than kl/+ (25.3 ± 0.7 g) and wild type (24.3 ± 0.5 g; P < 0.01 for each comparison). Serum FGF23 levels were >15-fold elevated in kl/kl and 3-fold elevated in kl/+ compared with wild type (Figure 6A). Serum calcitriol and phosphate levels were also significantly elevated in kl/kl compared with wild type but not in kl/+ (Figure 6A); there were no significant differences in PTH between the genotypes.

Representative pathology sections demonstrate LVH in kl/kl compared with wild type (Figure 6B). Although absolute left ventricular wall thickness was highest for kl/+ followed by kl/kl and then wild type (Figure 6C), the ratio of cardiac weight standardized to total body weight was highest in kl/kl (Figure 6D). When compared with wild type, kl/kl developed significantly increased relative wall thickness (Figure 6E), increased cross-sectional surface area of individual cardiac myocytes (Figure 6F), and changes in gene expression that are characteristic of LVH and similar to those we observed in NRVM (Figure 6G). Interestingly, morphological, immunocytochemical and mRNA analyses of kl/+ also reveal an LVH phenotype but in a pattern that is intermediate between kl/kl and wild type (Figure 6B-G). These results demonstrate a physiological model of elevated FGF23 levels in which LVH develops in a dose-dependent fashion.

FGFR activity is required for the development of LVH in a rat model of CKD Since the cardiac effects of FGF23 are mediated by FGFR activation and could be blocked in vitro by the FGFR inhibitor PD173074, we tested the hypothesis that PD173074 could also attenuate LVH in the 5/6 nephrectomy rat model of CKD, which is known to develop increased FGF23 levels, severe hypertension, and LVH. We performed 5/6 nephrectomy in male Sprague Dawley rats using standard surgical techniques, and administered by intraperitoneal injection either 1 mg/kg body weight/day of PD173074 dissolved in PBS (n=6) or vehicle alone (n=6) beginning one hour after surgery and continued daily for 14 days (Figure 7). As a negative control, 6 rats underwent sham nephrectomy. At day 14 after surgery, we measured blood pressure, performed echocardiograms, collected blood for laboratory testing, sacrificed the animals and examined their hearts. Immunoblotting revealed decreased hepatic expression of p-ERK protein levels in the rats that received PD173074 compared with the sham and 5/6 nephrectomized rats, indicating effective blockade of canonical FGFR-dependent MAPK signaling (data not shown). Renal function was significantly impaired and serum FGF23 levels significantly elevated in the animals that underwent 5/6 versus sham nephrectomy, but there were no differences between the 5/6 nephrectomized animals that received PD173074 or vehicle. Similarly, blood pressure was markedly increased in animals that underwent 5/6 versus sham nephrectomy, but no difference was detected between the 5/6 nephrectomy groups (data not shown). The 5/6 nephrectomized animals that received vehicle developed LVH compared with sham, but LVH was significantly attenuated in the animals that were injected with PD173074. Compared with vehicle, PD173074 treatment led to decreased left ventricular mass, reduced ratio of heart weight to total body weight, decreased left ventricular wall thickness and relative wall thickness, decreased cross-sectional surface area of individual cardiac myocytes, and increased left ventricular end diastolic volume and ejection fraction (Figure 7A-C). Compared with sham, 5/6 nephrectomy led to increased cardiac expression of f3-MHC and ANP and decreased expression of MCAD mRNA levels, each of which was not observed in PD173074-injected animals (data not shown). These data indicate that blocking FGFR attenuates the development of LVH in a well-established animal model of CKD with elevated FGF23 levels, despite having no effect on the severity of CKD or hypertension.

Example 2: Fibroblast Growth Factor Inhibition for the Treatment of Cardiovascular Disease

Cardiac effects of FGF23 are mediated by FGFR4. By using the pan-fibroblast growth factor receptor (FGFR) inhibitor PD173074 we have shown in Example 1 above that functional FGFRs in cardiac myocytes are necessary for the pro-hypertrophic effect of FGF23. Since these cells do not express klotho (Kuro-o M et al., Nature. 1997;390(6655):45-51), the receptor/coreceptor complex composition required for the induction of FGF23 signaling in cardiac myocytes must be different from the one present in classic FGF23 target cells. FGF23-induced LVH could be mediated by higher affinity binding to specific cardiac FGFRs, such as FGFR4, which have been previously detected in isolated cardiac myocytes and in total heart tissue (Faul C et al., J Clin Invest 2011; 121(11):4393-408; Hughes SE et al., J Histochem Cytochem. 1997;45(7):1005-19). It has been shown in in vitro binding studies that unlike FGFR1-3, FGFR4 is capable of binding FGF23 with high affinity even in the absence of klotho (Zhang X et al., J Biol Chem. 2006;281(23):15694-700). Therefore we hypothesize that FGF23-mediated binding to FGFR4 accounts for FGF23's klotho-independent activity in the heart.

To experimentally distinguish which of the four FGFR isoforms (FGFR1-4) present in cardiac myocytes mediates the FGF23 effect, we used FGFR decoy receptors (Fc-FGFR, R&D systems). These recombinant chimeric proteins, which consist of an extracellular FGFR domain and the Fc domain of a human immunoglobulin Gl, function as soluble FGF traps. We postulated that FGFR4 is a major component of the cardiac FGF23 receptor complex. In contrast, FGFR1, the main FGF2 receptor, does not bind FGF23 in vitro in the absence of klotho (Zhang et al., J Biol Chem. 2006 Jun 9;281(23):15694-700), and thus, we hypothesized that its decoy receptor (Fc-FGFR1) should not interfere with FGF23's cardiac effect.

Indeed, when we treated isolated neonatal rat ventricular myocytes (NRVM) with FGF23 in the presence of increasing concentrations of the FGFR4 decoy receptor (Fc-FGFR4), we observed a significant decrease in cell surface area, which was not as dramatic when we used Fc-FGFR1 (Figure 9A). In contrast, the FGF2-induced hypertrophic response was reduced in the presence of Fc-FGFR1, but not with Fc-FGFR4.

Next, we wanted to determine the effectiveness of this FGFR trapping system on FGF-induced LVH in mice. To our knowledge, Fc decoy receptors have not yet been used to study FGFR function in vivo in any biological context, but served as valuable tools to analyze the functions of other receptor tyrosine kinases, like receptors for vascular endothelial growth factor (VEGFR) and their role in tumor growth and metastasis. We used similar Fc-FGFR concentrations as described for Fc-VEGFR in animal models of cancer (Lin J et al., Cancer Res. 2005;65(15):6901-9). When we coinjected 40 µg/kg FGF23 together with 30 µg/kg Fc-FGFR4 or Fc-FGFR1 intravenously into mice ten times over a total period of five days as done before (as described above in Example 1), we detected a significant reduction of FGF23-induced LVH by Fc-FGFR4, but not by Fc-FGFR1, as determined by the analysis of cardiac morphology and myocyte surface area (Figure 9B-D).

These novel data indicate that FGF23 requires FGFR4, but not FGFR1, to induce its cardiac effect. All three endocrine FGFs (FGF19/21/23) have similar pathological effects on the heart. It has been shown in in vitro binding studies that FGFR4 is capable of not only binding FGF23 with high affinity in the absence of klotho (Zhang et al., J Biol Chem. 2006 Jun 9;281(23):15694-700), but also FGF19 and FGF21, the other members of the subfamily of endocrine FGFs (Harmer et al., Biochemistry. 2004 Jan 27;43(3):629-40). Therefore we hypothesize that all three members of the subfamily of endocrine FGFs have similar effects on the heart and that this effect is mediated by klotho-independent FGFR4 activation on cardiac myocytes.

When we treated NRVM with increasing concentrations of FGF19 or FGF21, using conditions which we have previously used to study the FGF2- and FGF23-mediated induction of cardiac hypertrophy in vitro (as described in Example 1 above), we observed a significant dose-dependent increase in cell surface area (Figure 10). In contrast, FGF4, an FGF family member known to mainly signal via FGFR1-3 (Zhang X et al., J Biol Chem. 2006;281(23):15694-700), did not induce a hypertrophic response.

When we injected 40 lug/kg FGF19 or FGF21 (Figure 11) into mice ten times over a total period of five days, as done before for FGF23 in Example 1 above, we detected a significant induction of LVH as determined by the analysis of cardiac morphology. In contrast, when FGF19 or FGF21 (Figure 11) were co-injected together with 30 lug/kg of the Fc-FGFR4 decoy receptor, mice were protected from developing LVH, an effect that we have previously observed for FGF23-mediated LVH (Figure 9B-D).

These novel data indicate that the three endocrine FGF family members - FGF19/21/23 - have similar effects on the heart, possibly by activating the same receptor (i.e. FGFR4) and downstream signaling pathways (i.e. calcineurin-NFAT).

### Example 3: Novel implications of the endocrine FGF-FGFR4 signaling pathway in the heart

By demonstrating direct pathological effects of FGF23 on the heart as described above in Example 1, our data reposition FGF23 from biomarker of risk, to mechanism of disease. Therefore the pharmacological interference with the cardiac FGF23 receptor in patients with CKD could be beneficial to prevent or attenuate the development of cardiorenal syndrome and Congestive Heart Failure (CHF). Two recent clinical studies have reported that FGF19 and FGF21 were significantly increased in the serum of 60 and 200 CKD patients, respectively (Reiche et al., Horm Metab Res. Mar;42(3): 178-81; Lin et al., PLoS One.6(4):e18398). In the case of FGF21, levels were independently associated with LVH in CKD (Lin et al., PLoS One.6(4):e18398). These findings suggest that FGF19 and FGF21, which are known FGFR4 ligands (Zhang et al., J Biol Chem. 2006 Jun 9;281(23):15694-700; Biochemistry. 2004 Jan 27;43(3):629-40), could have similar pathologcial effects on the heart as we have described for FGF23 in Example 1 above, and could therefore also function as disease mediators in uremic cardiomyopathy.

Diabetes mellitus, which affects an estimated 25.8 million people in the United States, is a well-recognized risk factor for developing heart failure, and cardiovascular disease (CVD) is the leading cause of diabetes-related morbidity and mortality. The Framingham Heart Study showed that the frequency of heart failure is twice and five times in diabetic men and women, respectively, compared to age-matched control subjects (Garcia MJ et al., Diabetes. 1974;23(2):105-11; Kannel WB et al., JAMA. 1979;241(19):2035-8). Increased incidence of heart failure in diabetic patients persisted despite correction for age, hypertension, obesity, hypercholesterolemia, and coronary artery disease. Diabetes is responsible for diverse cardiovascular complications such as increased atherosclerosis in large arteries and increased coronary atherosclerosis as well as pathological alterations in cardiac structure and function in the absence of changes in blood pressure and coronary artery disease, a condition called diabetic cardiomyopathy. Echocardiographic studies have shown that in diabetic cardiomyopathy, systolic dysfunction and LVH are important mechanisms of disease (Devereux et al., Circulation. 2000 May 16;101(19):2271-6; Struthers AD and Morris AD, Lancet. 2002 Apr 20;359(9315): 1430-2).

FGF21 has been described as an important metabolic factor that is able to correct multiple abnormal characteristics and to maintain metabolic parameters at "healthy" levels (Kharitonenkov A, Curr Opin Pharmacol. 2009;9(6):805-10). The novel data described herein indicate that in diabetic patients, many of whom have increased serum FGF21 levels (Chen WW et al., Exp Clin Endocrinol Diabetes. 2008;116(1):65-8.), elevated FGF21 by itself could induce LVH and thereby execute damaging effects on the heart. We hypothesize that FGF21 could be a circulating factor involved in the induction of diabetic cardiomyopathy, which might act independently of diabetes-induced kidney injury and accompanied high FGF23 serum levels. If so, interfering with the cardiac FGFR4 receptor in patients with diabetes might protect them from developing cardiomyopathy.

By analyzing FGF23-mediated cardiac hypertrophy in the background of CKD, we have illuminated a novel biological mechanism of LVH involving FGFR4. It is possible that agents blocking FGFR-induced LVH might have broad utility in patients with CKD and in the general population of non-CKD patients given the extremely high prevalence of LVH in general. Since common LVH therapeutics, such as anti-hypertensives, diuretics and "loosotropic" agents are only partially effective, targeting specific molecular mechanisms of disease will be necessary to improve the success of treatment. In this context, cardiac FGFR signaling regulated by endocrine FGFs could serve as a central mechanism regulating cardiac remodeling and therefore as a novel pharmacologcial target to prevent LVH. The fact that more than 5 million adults in the United States suffer from CHF, with costs to the American health care system of more than $37 billion, and that despite improved standards of care, the five-year moratlity rates following first admission for CHF approaches 50%, highlights the pressing need for new therpeutic approaches (Lloyd-Jones et al., Circulation. 2009 Jan 27;119(3):480-6).

## Claims

1. A composition for use in treating or preventing pathological left ventricular hypertrophy (LVH), or pathological LVH and congestive heart failure, the composition comprising a pharmaceutically acceptable carrier and an inhibitor of fibroblast growth factor receptor 4 (FGFR4) activity in an amount effective for inhibiting activation of FGFR4 and inhibiting signaling of at least one FGF selected from the group consisting of FGF19, FGF21, and FGF23.

2. The composition for use according to claim 1, wherein the at least one FGF is FGF23.

3. The composition for use according to claim 1, wherein the pathological LVH or pathological LVH and congestive heart failure is associated with chronic kidney disease (CKD).

4. The composition for use according to claim 1, wherein the composition is used in treating or preventing pathological LVH and congestive heart failure.

5. The composition for use according to claim 4, wherein the inhibitor is one selected from the group consisting of: an antisense nucleic acid, a shRNA, an siRNA, an antibody which binds to and inhibits activation or signaling of FGFR4, a small molecule which inhibits activation or signaling of FGFR4, and a chimeric protein comprising an extracellular domain of FGFR4 and an immunoglobulin Fc domain.

6. The composition for use according to claim 1, further comprising an inhibitor of the at least one FGF selected from the group consisting of FGF19, FGF21, and FGF23.

7. The composition for use according to claim 6, wherein the at least one FGF is FGF23.

8. The composition for use according to claim 6, wherein the inhibitor of the at least one FGF and the inhibitor of activation of FGFR4 are administered at the same time.

9. The composition for use according to claim 6, wherein the inhibitor of the at least one FGF and the inhibitor of activation of the FGFR4 are administered at different times.

10. The composition for use according to claim 6, wherein the inhibitor of the at least one FGF is one selected from the group consisting of: an antisense nucleic acid, a ribozyme, an antibody, a peptide or a peptidomimetic.

11. A method of determining whether a subject suffering from or at risk of pathological LVH or congestive heart failure is in need of inhibiting activation of FGFR4, comprising;
- determining if the level of at least one FGF selected from the group consisting of FGF19, FGF21, and FGF23 in a biological sample from the subject suffering from or at risk of pathological LVH or congestive heart failure is elevated compared to a normal level based on a database of subjects without pathological LVH or congestive heart failure and with normal blood levels of the at least one FGF; and
- correlating an increased level of the at least one FGF in the biological sample from the subject suffering from or at risk of pathological LVH or congestive heart failure relative to the normal level based on the database with a need for inhibiting activation of FGFR4 in the subject suffering from or at risk of pathological LVH or congestive heart failure.

12. The method of claim 11, wherein the subject is a human.

## Patentansprüche

1. Zusammensetzung zur Verwendung bei der Behandlung oder Vorbeugung von pathologischer linksventrikulärer Hypertrophie (LVH), oder pathologischer LVH und kongestiver Herzinsuffizienz, wobei die Zusammensetzung einen pharmazeutisch akzeptablen Träger und einen Inhibitor der Aktivität des Fibroblastenwachstumsfaktorrezeptors 4 (*Fibroblast Growth Factor Receptor 4* - FGFR4) in einer Menge, die wirksam ist zur Hemmung der Aktivierung von FGFR4 und Hemmung der Signalübertragung von mindestens einem FGF, ausgewählt aus der Gruppe bestehend aus FGF19, FGF21 und FGF23, umfasst.

2. Zusammensetzung zur Verwendung nach Anspruch 1, wobei der mindestens eine FGF FGF23 ist.

3. Zusammensetzung zur Verwendung nach Anspruch 1, wobei die pathologische LVH oder die pathologische LVH und kongestive Herzinsuffizienz mit chronischer Nierenerkrankung (CKD) verbunden sind.

4. Zusammensetzung zur Verwendung nach Anspruch 1, wobei die Zusammensetzung bei der Behandlung oder Vorbeugung von pathologischer LVH und kongestiver Herzinsuffizienz verwendet wird.

5. Zusammensetzung zur Verwendung nach Anspruch 4, wobei der Inhibitor eines ist, ausgewählt aus der Gruppe bestehend aus: einer Antisense-Nukleinsäure, einer shRNA, einer siRNA, einem Antikörper, der an FGFR4 bindet und die Aktivierung oder Signalübertragung von FGFR4 hemmt, einem kleinen Molekül, das die Aktivierung oder Signalübertragung von FGFR4 hemmt, und einem chimären Protein, das eine extrazelluläre Domäne von FGFR4 und eine Immunglobulin-Fc-Domäne umfasst.

6. Zusammensetzung zur Verwendung nach Anspruch 1, weiterhin umfassend einen Inhibitor des mindestens einen FGF, ausgewählt aus der Gruppe bestehend aus FGF19, FGF21 und FGF23.

7. Zusammensetzung zur Verwendung nach Anspruch 6, wobei der mindestens eine FGF FGF23 ist.

8. Zusammensetzung zur Verwendung nach Anspruch 6, wobei der Inhibitor des mindestens einen FGF und der Inhibitor der Aktivierung von FGFR4 gleichzeitig verabreicht werden.

9. Zusammensetzung zur Verwendung nach Anspruch 6, wobei der Inhibitor des mindestens einen FGF und der Inhibitor der Aktivierung des FGFR4 zu unterschiedlichen Zeiten verabreicht werden.

10. Zusammensetzung zur Verwendung nach Anspruch 6, wobei der Inhibitor des mindestens einen FGF eines ist, ausgewählt aus der Gruppe bestehend aus: einer Antisense-Nukleinsäure, einem Ribozym, einem Antikörper, einem Peptid oder einem Peptidomimetikum.

11. Verfahren zur Feststellung, ob ein Individuum, das an pathologischer LVH oder kongestiver Herzinsuffizienz leidet oder ein Risiko dafür aufweist, eine Hemmung der Aktivierung von FGFR4 benötigt, umfassend:
- Feststellen, ob der Wert von mindestens einem FGF, ausgewählt aus der Gruppe bestehend aus FGF19, FGF21 und FGF23, in einer biologischen Probe von dem Individuum, das an pathologischer LVH oder kongestiver Herzinsuffizienz leidet oder ein Risiko dafür aufweist, gegenüber einem Normalwert, basierend auf einer Datenbank von Individuen ohne pathologische LVH oder kongestive Herzinsuffizienz und mit normalen Blutwerten des mindestens einen FGF, erhöht ist; und
- Korrelieren eines erhöhten Wertes des mindestens einen FGF in der biologischen Probe von dem Individuum, das an pathologischer LVH oder kongestiver Herzinsuffizienz leidet oder ein Risiko dafür aufweist, im Verhältnis zu dem auf der Datenbank basierenden Normalwert mit einer Notwendigkeit der Hemmung der Aktivierung von FGFR4 bei dem Individuum, das an pathologischer LVH oder kongestiver Herzinsuffizienz leidet oder ein Risiko dafür aufweist.

12. Verfahren nach Anspruch 11, wobei das Individuum ein Mensch ist.

## Revendications

1. Composition destinée à une utilisation pour le traitement ou la prévention d'une l'hypertrophie ventriculaire gauche pathologique (*Left Ventricular Hypertrophy* - LVH), ou d'une LVH pathologique et d'une insuffisance cardiaque congestive, ladite composition comprenant un véhicule pharmaceutiquement acceptable et un inhibiteur de l'activité du récepteur 4 du facteur de croissance des fibroblastes (*Fibroblast Growth Factor Receptor 4* - FGFR4) dans une teneur efficace pour inhiber l'activation du FGFR4 et inhiber la signalisation d'au moins un FGF sélectionné dans le groupe comprenant FGF19, FGF21 et FGF23.

2. Composition destinée à une utilisation selon la revendication 1, où ledit au moins un FGF est le FGF23.

3. Composition destinée à une utilisation selon la revendication 1, où la LVH pathologique ou la LVH pathologique et l'insuffisance cardiaque congestive sont associées à une insuffisance rénale chronique (IRC).

4. Composition destinée à une utilisation selon la revendication 1, où ladite composition est utilisée pour le traitement ou la prévention d'une LVH pathologique et d'une insuffisance cardiaque congestive.

5. Composition destinée à une utilisation selon la revendication 4, où l'inhibiteur est un inhibiteur sélectionné dans le groupe comprenant : un acide nucléique antisens, un shRNA, un siRNA, un anticorps se liant à, et inhibant l'activation ou la signalisation du FGFR4, une petite molécule inhibant l'activation ou la signalisation du FGFR4, et une protéine chimérique comprenant un domaine extracellulaire du FGFR4 et un domaine immunoglobuline Fc.

6. Composition destinée à une utilisation selon la revendication 1, comprenant en outre un inhibiteur dudit au moins un FGF sélectionné dans le groupe comprenant FGF19, FGF21 et FGF23.

7. Composition destinée à une utilisation selon la revendication 6, où ledit au moins un FGF est le FGF23.

8. Composition destinée à une utilisation selon la revendication 6, où l'inhibiteur dudit au moins un FGF et l'inhibiteur de l'activation du FGFR4 sont administrés en même temps.

9. Composition destinée à une utilisation selon la revendication 6, où l'inhibiteur dudit au moins un FGF et l'inhibiteur de l'activation du FGFR4 sont administrés à des moments différents.

10. Composition destinée à une utilisation selon la revendication 6, où l'inhibiteur dudit au moins un FGF est un inhibiteur sélectionné dans le groupe comprenant : un acide nucléique antisens, un ribozyme, un anticorps, un peptide ou un peptidomimétique.

11. Procédé de détermination si un sujet souffrant, ou présentant un risque de LVH pathologique ou d'insuffisance cardiaque congestive a besoin d'une inhibition d'activation du FGFR4, comprenant :
- la détermination si le niveau d'au moins un FGF sélectionné dans le groupe comprenant FGF19, FGF21 et FGF23 dans un échantillon biologique du sujet souffrant, ou présentant un risque de LVH pathologique ou d'insuffisance cardiaque congestive, est élevé par rapport à un niveau normal provenant d'une base de données de sujets sans LVH pathologique ni insuffisance cardiaque congestive et avec des niveaux sanguins normaux dudit au moins un FGF; et
- la corrélation d'un niveau plus élevé dudit au moins un FGF dans l'échantillon biologique du sujet souffrant, ou présentant un risque de LVH pathologique ou d'insuffisance cardiaque congestive, par rapport au niveau normal provenant de la base de données, avec un besoin d'inhibition d'activation du FGFR4 chez le sujet souffrant, ou présentant un risque de LVH pathologique ou d'insuffisance cardiaque congestive.

12. Procédé selon la revendication 11, où le sujet est humain.


### SEQUENCE LISTING

<110> UNIVERSITY OF MIAMI Wolf, Myles Faul, Christian
<120> FIBROBLAST GROWTH FACTOR RECEPTOR INHIBITION FOR THE TREATMENT OF DISEASE
<130> 7230-164WO
<150> US 61/501,025
   <151> 2011-06-24
<160> 26
<170> PatentIn version 3.5
<210> 1
   <211> 62
   <212> DNA
   <213> ARTIFICIAL SEQUENCE
<220>
   <223> SYNTHETIC CONSTRUCT
<400> 1
   agctaaaaag ccgtcaagat gctcaaagac tctcttgaag tctttgagca tcttgacggc 60
   gg 62
<210> 2
   <211> 19
   <212> RNA
   <213> ARTIFICIAL SEQUENCE
<220>
   <223> SYNTHETIC CONSTRUCT
<400> 2
   ggcucuuccg gcaagucaa 19
<210> 3
   <211> 20
   <212> DNA
   <213> ARTIFICIAL SEQUENCE
<220>
   <223> OLIGONUCLEOTIDE
<400> 3
   cttcacagca gaggagaagg 20
<210> 4
   <211> 20
   <212> DNA
   <213> ARTIFICIAL SEQUENCE
<220>
   <223> OLIGONUCLEOTIDE
<400> 4
   acacctgctg tacactctgc 20
<210> 5
   <211> 20
   <212> DNA
   <213> ARTIFICIAL SEQUENCE
<220>
   <223> OLIGONUCLEOTIDE
<400> 5
   ctccagaaga gaagaactcc 20
<210> 6
   <211> 20
   <212> DNA
   <213> ARTIFICIAL SEQUENCE
<220>
   <223> OLIGONUCLEOTIDE
<400> 6
   ccacctgctg gacattctgc 20
<210> 7
   <211> 20
   <212> DNA
   <213> ARTIFICIAL SEQUENCE
<220>
   <223> OLIGONUCLEOTIDE
<400> 7
   agcgagcaga ccgatgaagc 20
<210> 8
   <211> 20
   <212> DNA
   <213> ARTIFICIAL SEQUENCE
<220>
   <223> OLIGONUCLEOTIDE
<400> 8
   agcagcttga ccttcgcagg 20
<210> 9
   <211> 20
   <212> DNA
   <213> ARTIFICIAL SEQUENCE
<220>
   <223> OLIGONUCLEOTIDE
<400> 9
   ccagatgatt ctgctcctgc 20
<210> 10
   <211> 20
   <212> DNA
   <213> ARTIFICIAL SEQUENCE
<220>
   <223> OLIGONUCLEOTIDE
<400> 10
   tgaactatgt gccatcttgg 20
<210> 11
   <211> 19
   <212> DNA
   <213> ARTIFICIAL SEQUENCE
<220>
   <223> OLIGONUCLEOTIDE
<400> 11
   cggtgctctg acaccagag 19
<210> 12
   <211> 20
   <212> DNA
   <213> ARTIFICIAL SEQUENCE
<220>
   <223> OLIGONUCLEOTIDE
<400> 12
   agaggcaaag tacgtgttcc 20
<210> 13
   <211> 20
   <212> DNA
   <213> ARTIFICIAL SEQUENCE
<220>
   <223> OLIGONUCLEOTIDE
<400> 13
   tgccagctgc caagacggtg 20
<210> 14
   <211> 20
   <212> DNA
   <213> ARTIFICIAL SEQUENCE
<220>
   <223> OLIGONUCLEOTIDE
<400> 14
   aaggatgggc cggtgagggg 20
<210> 15
   <211> 20
   <212> DNA
   <213> ARTIFICIAL SEQUENCE
<220>
   <223> OLIGONUCLEOTIDE
<400> 15
   gctccatgct gtccctgccg 20
<210> 16
   <211> 20
   <212> DNA
   <213> ARTIFICIAL SEQUENCE
<220>
   <223> OLIGONUCLEOTIDE
<400> 16
   tccccgagtg cttcaggacc 20
<210> 17
   <211> 20
   <212> DNA
   <213> ARTIFICIAL SEQUENCE
<220>
   <223> OLIGONUCLEOTIDE
<400> 17
   agtgttctgc gtggcggtcg 20
<210> 18
   <211> 20
   <212> DNA
   <213> ARTIFICIAL SEQUENCE
<220>
   <223> OLIGONUCLEOTIDE
<400> 18
   gcacagcaca cgccgggtta 20
<210> 19
   <211> 20
   <212> DNA
   <213> ARTIFICIAL SEQUENCE
<220>
   <223> OLIGONUCLEOTIDE
<400> 19
   ggctatgctg tggccgcact 20
<210> 20
   <211> 20
   <212> DNA
   <213> ARTIFICIAL SEQUENCE
<220>
   <223> OLIGONUCLEOTIDE
<400> 20
   ggtctgaggg caccacgctc 20
<210> 21
   <211> 22
   <212> DNA
   <213> ARTIFICIAL SEQUENCE
<220>
   <223> OLIGONUCLEOTIDE
<400> 21
   gactttctga gtcaggacaa gg 22
<210> 22
   <211> 22
   <212> DNA
   <213> ARTIFICIAL SEQUENCE
<220>
   <223> OLIGONUCLEOTIDE
<400> 22
   gttacccaga ggcaagatca gg 22
<210> 23
   <211> 20
   <212> DNA
   <213> ARTIFICIAL SEQUENCE
<220>
   <223> OLIGONUCLEOTIDE
<400> 23
   gtcttcggcc ttgttctacc 20
<210> 24
   <211> 20
   <212> DNA
   <213> ARTIFICIAL SEQUENCE
<220>
   <223> OLIGONUCLEOTIDE
<400> 24
   cgaagtaagg ttatctgagg 20
<210> 25
   <211> 20
   <212> DNA
   <213> ARTIFICIAL SEQUENCE
<220>
   <223> OLIGONUCLEOTIDE
<400> 25
   tatgtcgtgg agtctactgg 20
<210> 26
   <211> 20
   <212> DNA
   <213> ARTIFICIAL SEQUENCE
<220>
   <223> OLIGONUCLEOTIDE
<400> 26
   agtgatggca tggactgtgg 20